(19) Europäisches Patentamt
European Patent Office
Office européen des brevets

(11) EP 3 805 327 B1

(12) **EUROPEAN PATENT SPECIFICATION**

(45) Date of publication and mention of the grant of the patent:
**15.04.2026 Bulletin 2026/16**

(21) Application number: **19812177.4**

(22) Date of filing: **28.05.2019**

(51) International Patent Classification (IPC):
***C09D 5/16*** *(2006.01)* ***C09D 201/02*** *(2006.01)*
***C09D 7/63*** *(2018.01)* ***C08F 220/10*** *(2006.01)*
***C08F 230/08*** *(2006.01)* *C09F 1/04 (2006.01)*
*C07C 41/54 (2006.01)* *C07C 43/303 (2006.01)*
*C07C 67/04 (2006.01)* *C07C 69/54 (2006.01)*
*C07C 69/753 (2006.01)* *C07C 69/22 (2006.01)*
*C08K 5/101 (2006.01)* *C08K 5/06 (2006.01)*
*C08F 8/14 (2006.01)* *C08F 220/18 (2006.01)*
*C08F 220/28 (2006.01)* *C08F 220/14 (2006.01)*
*C08F 220/06 (2006.01)* *C09D 133/14 (2006.01)*
*C09D 143/04 (2006.01)* *C09D 193/04 (2006.01)*
*C08L 33/14 (2006.01)* *C08L 43/04 (2006.01)*

(52) Cooperative Patent Classification (CPC):
(C-Sets available)
**C09D 5/1668; C07C 67/04; C08F 8/14; C08F 220/14; C08F 220/1802; C08F 220/1806; C09D 5/1618; C09D 5/1625; C09D 5/1687; C09D 7/63; C09D 133/14; C09D 143/04; C09D 193/04; C09F 1/04;** C08F 2800/20; (Cont.)

(86) International application number:
**PCT/JP2019/020988**

(87) International publication number:
**WO 2019/230675 (05.12.2019 Gazette 2019/49)**

(54) **ANTIFOULING PAINT COMPOSITION**

NICHT FAULENDE FARBZUSAMMENSETZUNG

COMPOSITION DE PEINTURE ANTISALISSURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2018 JP 2018105921**

(43) Date of publication of application:
**14.04.2021 Bulletin 2021/15**

(73) Proprietor: **Mitsubishi Chemical Corporation**
**Chiyoda-ku**
**Tokyo 100-8251 (JP)**

(72) Inventors:
- **KATSUMATA, Sho**
  **Tokyo 100-8251 (JP)**
- **TANIGUCHI, Kana**
  **Tokyo 100-8251 (JP)**
- **NAKAMURA, Junichi**
  **Tokyo 100-8251 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 439 025** **EP-A1- 1 695 956**
**EP-A1- 3 284 783** **WO-A1-2016/167360**
**WO-A1-2017/065172** **WO-A1-2018/008166**
**WO-A1-2018/181429** **JP-A- 2001 261 620**
**JP-A- 2001 262 076** **JP-A- 2001 262 076**
**JP-A- 2008 106 047** **JP-A- 2010 100 821**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**JP-A- 2018 062 555** **JP-A- H06 135 877**

(52) Cooperative Patent Classification (CPC): (Cont.)
C08F 2810/50; C08K 5/101; C08K 5/103

C-Sets
**C07C 67/04, C07C 69/54;**
**C08F 8/14, C08F 220/1802;**
**C08F 220/1802, C08F 220/281, C08F 220/14, C08F 220/281;**
**C08F 220/1806, C08F 220/281, C08F 220/1802, C08F 220/14;**
**C09D 133/14, C08K 5/101;**
**C09D 133/14, C08L 93/04;**
**C09D 133/14, C08L 93/04, C08K 5/06;**
**C09D 143/04, C08L 93/04;**
**C09D 143/04, C08L 93/04, C08K 5/06;**
**C09D 193/04, C08K 5/06, C08L 33/04;**
C08F 220/14, C08F 230/085, C08F 220/1802, C08F 220/281;
C08F 220/1802, C08F 220/14, C08F 220/281, C08F 220/06;
C08F 220/1802, C08F 220/14, C08F 220/281, C08F 220/281;
C08F 220/1802, C08F 230/085, C08F 220/281, C08F 220/14, C08F 220/281

## Description

[Technical Field]

The present invention relates to an antifouling paint composition.
Priority is claimed on Japanese Patent Application No. 2018-105921, filed on June 1, 2018.

[Background Art]

It is known that marine structures or ships are coated with antifouling paints in order to prevent adhesion of marine organisms that cause corrosion of a portion in contact with sea water or a decrease in sailing speed.
Self-polishing antifouling paints are known as antifouling paints. In a case of a coating film obtained from the self-polishing antifouling paints, a surface of the coating film is gradually dissolved in sea water to renew the surface (self-polished), and the surface of the coating film is constantly exposed to antifouling components. Therefore, an antifouling effect is exhibited for a long period of time.
As the self-polishing antifouling paint, a hydrolyzable resin is used. Examples thereof include a vinyl polymer having a hemiacetal ester group or a hemiketal ester group in a side chain (Patent Literature 1) and a vinyl polymer having an organic silyl group. In addition, there is rosin blended with these hydrolyzable resins in order to improve antifouling property or improve solubility (Patent Literatures 2 to 5). Further, in recent years, reduction of a volatile organic compound (hereinafter, also referred to as a "VOC") becomes important due to the impact on the environment or the like, and reduction of the VOC has also been examined for the self-polishing antifouling paint.
Patent Literature 6 describes a resin composition for producing a low-VOC antifouling coating composition, wherein the resin composition has a polymer comprising a monomer-derived structural unit and after the resin composition is stored at 40°C for 30 days, the decomposition rate of the structure in the polymer is 20% or lower.
Patent Literature 7 relates to a (meth)acrylic copolymer, a resin composition, an antifouling coating composition and a method for producing a (meth)acrylic copolymer.
Patent Literature 8 is related to an antifouling coating composition, which can form a coating film and a polymer-containing composition which is suitable for obtaining the antifouling coating composition.
Patent Literature 9 relates to a rosin derivative highly soluble in organic solvents in general, compatible with various kinds of resins, and suitable for use in soldering flux or soldering paste for soldering parts to curable compositions, and packaging substrates.
Patent Literature 10 relates to a rosin derivative suitable for a curable composition.
Patent Literature 11 relates to a resin composition comprising a (meth)acrylic copolymer, a method for producing the same, and an antifouling paint composition.
Patent Literature 12 relates to a novel rosin derivative having a (meth) acryloyl group and a method for producing the same.
Patent Literature 13 relates to a (meth) acrylic copolymer, a resin composition, an antifouling coating composition and a process for producing a (meth) acrylic copolymer.
Patent Literature 14 relates to a novel rosin copolymer, an active energy ray-curable resin composition containing the same, and a cured product thereof.
Patent Literature 15 relates to a 1-substituted ethyl ester which is useful for coating resins or photosensitive materials and the synthesis thereof.
Patent Literature 16 relates to a novel cycloalkenylcarboxylic acid, a bicycloalkenylcarboxylic acid, derivatives thereof, a compounding agent for antifouling paint which comprises said acid or derivative, an antifouling paint composition, an antifouling coating film, a ship, an underwater structure, a fishing tackle or a fishing net coated with the antifouling coating film, and an antifouling method for them.
Patent Literature 17 relates to soldering flux compositions, solder pastes, and methods of soldering using the same.

[Citation List]

[Patent Literature]

[Patent Literature 1]
Japanese Unexamined Patent Application, First Publication No. H4-103671
[Patent Literature 2]

Japanese Unexamined Patent Application, First Publication No. H10-30071
[Patent Literature 3]
Japanese Unexamined Patent Application, First Publication No. H11-116858
[Patent Literature 4]
Japanese Unexamined Patent Application, First Publication No. 2001-226440
[Patent Literature 5]
Japanese Unexamined Patent Application, First Publication No. 2005-082725
[Patent Literature 6]
EP 3 284 783 A1
[Patent Literature 7]
WO 2017/065172 A1
[Patent Literature 8]
WO 2018/008166 A1
[Patent Literature 9]
JP 2001-262076 A
[Patent Literature 10]
JP 2001-261620 A
[Patent Literature 11]
WO 2018/181429 A1
[Patent Literature 12]
JP 2008-106047 A
[Patent Literature 13]
JP 2018-062555 A
[Patent Literature 14]
JP 2010-100821 A
[Patent Literature 15]
JP H06-135877 A
[Patent Literature 16]
EP 1 695 956 A1
[Patent Literature 17]
EP 1 439 025 A1

[Summary of Invention]

[Technical Problem]

In order to decrease a VOC content, it is necessary to reduce a solvent content. However, the viscosity increases due to an increase in solid content, and thus it becomes difficult to prepare or apply the antifouling paint.

In a case where the solvent content is reduced, paint viscosity is high and coatability is poor in Patent Literatures 1 to 5. Furthermore, the coating film of the antifouling paint using the vinyl polymer described in Patent Literature 1 does not have sufficient antifouling property. In addition, the coating film of the antifouling paint using a (meth)acrylic copolymer described in Patent Literatures 2 to 5 has problems in that water resistance is low and cracks occur over time. Patent Literature 3 has a problem in that storage stability of the paint is low.

An object of the present invention is to provide an antifouling paint composition which can form a coating film having excellent antifouling property and water resistance, has good storage stability, and has a low viscosity and good coatability when a VOC content is reduced.

[Solution to Problem]

The present invention is defined in the appended claims and relates to the following embodiments.

The present invention provides an antifouling paint composition, comprising: at least one compound (A) selected from the group consisting of compounds represented by Formula (1), Formula (2), and Formula (3); and a vinyl-based copolymer (B), wherein the content of the compound (A) in the antifouling paint composition is 3% by mass or greater, and the mass ratio of the content of the compound (A) to the vinyl-based copolymer (B) in the antifouling paint composition is 0.1/99.9 to 70/30.

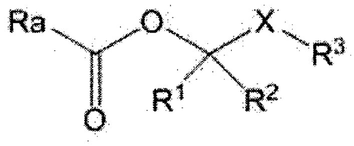
(1)
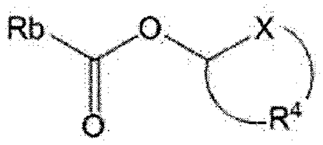
(2)
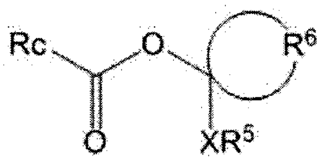
(3)

(in the formulae, Ra, Rb, and Rc represent a hydrocarbon group having 1 to 40 carbon atoms; X represents -O-, -S-, or $-NR^{14}-$, where $R^{14}$ represents a hydrogen atom or an alkyl group; $R^1$ and $R^2$ each represents a hydrogen atom or an alkyl group having 1 to 10 carbon atoms; $R^3$ and $R^5$ each represents an alkyl group having 1 to 20 carbon atoms, a cycloalkyl group, or an aryl group; and $R^4$ and $R^6$ each represents an alkylene group having 1 to 10 carbon atoms).

In one embodiment, Ra, Rb, or Rc of the compound (A) is a cyclic hydrocarbon residue derived from rosin.

In one embodiment, the vinyl-based copolymer (B) is a (meth)acrylic copolymer including at least one constituent unit selected from the group consisting of a constituent unit (u1) having at least one structure (I) represented by Formula (4), Formula (5), or Formula (6), a constituent unit (u2) containing a triorganosilyloxycarbonyl group, and a constituent unit (u3) having at least one structure (III) represented by Formula (7) or Formula (8),

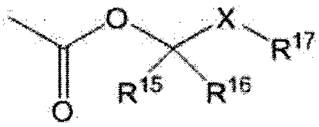
(4)
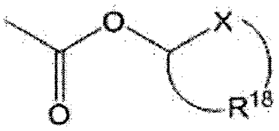
(5)
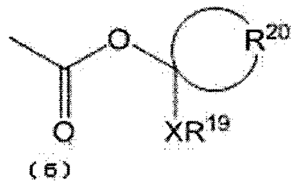
(6)

(in the formulae, X represents -O-, -S-, or $-NR^{21}-$, where $R^{21}$ represents a hydrogen atom or an alkyl group; $R^{15}$ and $R^{16}$ each represents a hydrogen atom or an alkyl group having 1 to 10 carbon atoms; $R^{17}$ and $R^{19}$ each represents an alkyl group having 1 to 20 carbon atoms, a cycloalkyl group, or an aryl group; and $R^{18}$ and $R^{20}$ each represents an alkylene group having 1 to 10 carbon atoms),

$$-COO-M-OCO \cdots \quad (7)$$

$$-COO-M-R^{13} \cdots \quad (8)$$

(in the formulae, M represents Zn, Cu, Mg, or Ca; and $R^{13}$ represents an organic acid residue other than a (meth) acryloyloxy group).

In one embodiment, the antifouling paint composition further comprises: at least one selected from the group consisting of a compound that reacts with an acid, a basic compound, an acidic compound, and a dehydrating agent.

In one embodiment, the compound that reacts with an acid is at least one compound (Y3) selected from the group consisting of compounds represented by Formula (31), Formula (32), and Formula (33),

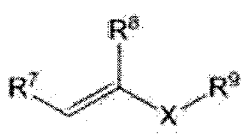
(31)
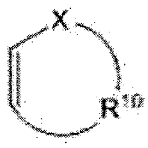
(32)
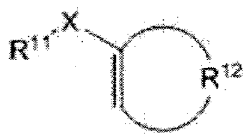
(33)

(in the formulae, X represents -O-, -S-, or $-NR^{21}-$, where $R^{21}$ represents a hydrogen atom or an alkyl group; $R^7$ represents a hydrogen atom or an alkyl group having 1 to 9 carbon atoms; $R^8$ represents a hydrogen atom or an alkyl group having 1 to 10 carbon atoms; $R^9$ and $R^{11}$ each represents an alkyl group having 1 to 20 carbon atoms, a cycloalkyl group, or an aryl group; $R^{10}$ represents a single bond or an alkylene group having 1 to 9 carbon atoms; and $R^{12}$ represents an alkylene group having 1 to 9 carbon atoms).

In one embodiment, the antifouling paint composition further comprises an antifouling agent.

In one embodiment, the antifouling paint composition contains, as the antifouling agent, at least one selected from the group consisting of cuprous oxide, pyridine triphenyl borane, 4,5-dichloro-2-n-octyl-4-isothiazolin-3-one, 4-bromo-2-(4-chlorophenyl)-5-(trifluoromethyl)-1H-pyrrole-3-carbonitrile, and medetomidine.

[Advantageous Effects of Invention]

According to the present invention, it is possible to provide an antifouling paint composition which can form a coating film having excellent antifouling property and water resistance, has good storage stability, and has good coatability when a VOC content is reduced.

[Description of Embodiments]

The definitions of the following terms apply throughout the present specification and claims.

A "cyclic hydrocarbon residue derived from rosin" refers to a remaining cyclic hydrocarbon group, in a cyclic hydrocarbon skeleton of various resin acids contained in rosin used as a raw material, excluding one or more carboxyl groups present on the skeleton.

A "volatile organic compound (VOC)" refers to an organic compound which easily volatilizes at room temperature under normal pressure. Further, room temperature refers to a temperature range of 10°C to 30°C and normal pressure refers to a pressure range of 1000 Pa to 1050 Pa.

[Compound (A)]

An antifouling paint composition of the present invention comprises: at least one compound (A) selected from the group consisting of compounds represented by Formula (1), Formula (2), and Formula (3); and a vinyl-based copolymer (B), wherein the content of the compound (A) in the antifouling paint composition is 3% by mass or greater, and the mass ratio of the content of the compound (A) to the vinyl-based copolymer (B) in the antifouling paint composition is 0.1/99.9 to 70/30.

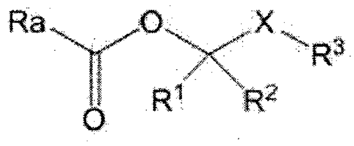

(1)

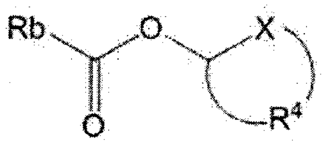

(2)

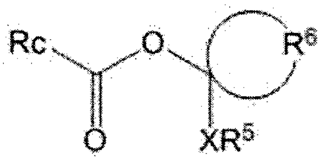

(3)

In the formulae above, Ra, Rb, and Rc represent a hydrocarbon group having 1 to 40 carbon atoms; X represents -O-, -S-, or -$NR^{14}$-, where $R^{14}$ represents a hydrogen atom or an alkyl group; $R^1$ and $R^2$ each represents a hydrogen atom or an alkyl group having 1 to 10 carbon atoms; $R^3$ and $R^5$ each represents an alkyl group having 1 to 20 carbon atoms, a cycloalkyl group, or an aryl group; and $R^4$ and $R^6$ each represents an alkylene group having 1 to 10 carbon atoms.

In Formulae (1) to (3), examples of the hydrocarbon group of Ra, Rb, and Rc include a linear hydrocarbon group, a branched hydrocarbon group, and a cyclic hydrocarbon group. Examples of the linear hydrocarbon group and branched hydrocarbon group include alkyl groups such as a methyl group, an ethyl group, a propyl group, an isopropyl group, a butyl group, an isobutyl group, a pentyl group, a hexyl group, a 2-ethylhexyl group, a lauryl group, and a stearyl group. In addition, a hydrocarbon residue derived from a fatty acid such as linoleic acid or versatic acid may be used, and a hydrocarbon residue derived from versatic acid is preferably used. The number of carbon atoms is 1 to 40, preferably 1 to 20, more preferably 3 to 18, and even more preferably 5 to 15.

Examples of the cyclic hydrocarbon group include a cyclohexyl group, a cyclopentyl group, a phenyl group, a naphthyl group, and a hydrocarbon residue derived from a carboxylic acid such as trimethylisobutenylcyclohexenecarboxylic acid and salicylic acid, and a cyclic hydrocarbon residue derived from rosin.

As the hydrocarbon groups of Ra, Rb, and Rc, a cyclic hydrocarbon group is more preferable from the viewpoint of antifouling property, water resistance, and physical property of a coating film. The cyclic hydrocarbon residue derived from rosin is most preferable. Examples of the rosin include rosin to be described below.

In Formulae (1) to (3), X may represent any of -O- (an etheric oxygen atom), -S- (a sulfide-based sulfur atom), or -$NR^{14}$-, and it is preferable that X represent -O-.

In Formula (1), examples of the alkyl group having 1 to 10 carbon atoms as $R^1$ and $R^2$ include a methyl group, an ethyl group, a propyl group, an isopropyl group, a butyl group, an isobutyl group, a pentyl group, a hexyl group, and a 2-ethylhexyl group.

The number of carbon atoms in the alkyl group as $R^1$ and $R^2$ is preferably in a range of 1 to 4, more preferably in a range of 1 to 3, and still more preferably 1 or 2.

Preferred examples of a combination of $R^1$ and $R^2$ include a combination of a hydrogen atom and a methyl group, a combination of a methyl group and a methyl group, a combination of a hydrogen atom and an alkyl group having 2 to 10

carbon atoms (hereinafter, also referred to as a "long chain alkyl group"), a combination of a methyl group and a long chain alkyl group, a combination of a hydrogen atom and a hydrogen atom, and a combination of a long chain alkyl group and a long chain alkyl group. Among these, from the viewpoint of the hydrolyzability, the combination of a hydrogen atom and a methyl group is preferable.

Examples of the alkyl group having 1 to 20 carbon atoms as $R^3$ include the alkyl groups exemplified as the alkyl group having 1 to 10 carbon atoms above, a decyl group, a dodecyl group, and a tetradecyl group. The number of carbon atoms in the alkyl group as $R^3$ is preferably in a range of 1 to 10.

As the cycloalkyl group, a cycloalkyl group having 4 to 8 carbon atoms is preferable, and examples thereof include a cyclohexyl group and a cyclopentyl group.

As the aryl group, an aryl group having 6 to 20 carbon atoms is preferable, and examples thereof include a phenyl group and a naphthyl group.

It is preferable that $R^3$ represent an alkyl group having 1 to 10 carbon atoms or a cycloalkyl group.

The alkyl group, the cycloalkyl group, or the aryl group may be substituted with a substituent selected from the group consisting of a cycloalkyl group, an aryl group, an alkoxy group, an alkanoyloxy group, an aralkyl group, and an acetoxy group. In a case where the group is substituted with a substituent, the number of substituents may be one or may be two or more.

Examples of the cycloalkyl group and the aryl group as a substituent are the same as those described above. Examples of the alkoxy group include a methoxy group, an ethoxy group, a propoxy group, and a butoxy group. Examples of the alkanoyloxy group include an ethanoyloxy group. Examples of the aralkyl group include a benzyl group.

In Formula (2), examples of the alkylene group having 1 to 10 carbon atoms as $R^4$ include a methylene group, an ethylene group, a propylene group, a butylene group, and a hexylene group.

The number of carbon atoms in the alkylene group as $R^4$ is preferably in a range of 2 to 7 and more preferably 3 or 4.

The alkylene group may be substituted with a substituent selected from the group consisting of a cycloalkyl group, an aryl group, an alkoxyl group, an alkanoyloxy group, an aralkyl group, and an acetoxy group. In a case where the group is substituted with a substituent, the number of substituents may be one or may be two or more. Specific examples of the substituent with which the alkylene group may be substituted are the same as those exemplified as the substituent in $R^3$.

In Formula (3), $R^5$ has the same definition as that for $R^3$ in Formula (1), and the preferable aspects thereof are also the same as described above.

$R^6$ has the same definition as that for $R^4$ in Formula (2), and the preferable aspects thereof are also the same as described above.

The compound (A) is a hydrolyzable compound in which a carboxy group is protected, and can suppress cross-linking with a metal element derived from copper or zinc in a paint and suppress paint viscosity as compared with a compound without protection, and has excellent coatability. In addition, when a large amount of rosins or monocarboxylic acid compounds that do not protect the carboxy group are blended, the antifouling property tends to be improved. However, since water gets inside the coating film, the water resistance decreases and cracks are generated. The compound (A) protected with a carboxy group can suppress the permeation of water into the coating film, and thus has excellent water resistance and long-term antifouling property.

The compound (A) can be synthesized by reacting a carboxy group of a compound (A0) having a carboxy group with a compound (Y1).

Examples of the compound (A0) include naphthenic acid, trimethylisobutenyl cyclohexenecarboxylic acid, stearic acid, salicylic acid, linoleic acid, versatic acid, and the like, or rosins such as gum rosin, wood rosin, tall oil rosin, hydrogenated rosin, and disproportionated rosin.

As the rosin, known rosins can be used. Examples thereof can include natural rosin (such as gum rosin, tall oil rosin, and wood rosin); rosin obtained by stabilizing the natural rosin by a known method (such as hydrogenated rosin, and disproportionated rosin); and various modified rosins using the natural rosin (such as rosin obtained by Diels-Alder reaction of $\alpha,\beta$ unsaturated carboxylic acids such as acrylic acid, methacrylic acid, itaconic acid, maleic(anhydride) acid, and fumaric acid (referred to as $\alpha,\beta$ unsaturated carboxylic acid modified rosin) and polymerized rosin). As the rosin, the natural rosin, the hydrogenated rosin, or the disproportionated rosin is preferable.

The rosin may contain resin acids described below, and may also contain impurities, foreign substances, and the like. In the present invention, rosins purified by a known method (for example, a recrystallization method) can also be used.

Examples of the resin acid contained in the rosin include monobasic resin acids (such as abietic acid, neoabietic acid, palustric acid, levopimaric acid, dehydroabietic acid, dihydroabietic acid, tetrahydroabietic acid, pimaric acid, isopimaric acid, and sandaracopimaric acid); dibasic resin acid (such as a dimer of the monobasic resin acid, and acrylopimaric acid); tribasic resin acid (such as fumaric malic acid); and monobasic resin acids having an anhydrous ring (such as maleo-pimelic acid), other polybasic resin acids (such as each dimer of acrylopimaric acid, maleo-pimelic acid, and fumaric malic acid), and resin acids obtained by hydrogenating these.

Examples of the compound (Y1) include compounds represented by Formula (31), Formula (32), and Formula

(33).

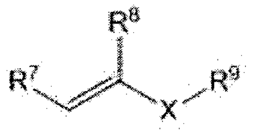
(31)

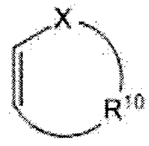
(32)

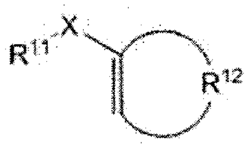
(33)

In the formulae above, X represents -O-, -S-, or $-NR^{14}-$, where $R^{14}$ represents a hydrogen atom or an alkyl group; $R^7$ represents a hydrogen atom or an alkyl group having 1 to 9 carbon atoms; $R^8$ represents a hydrogen atom or an alkyl group having 1 to 10 carbon atoms; $R^9$ and $R^{11}$ each represents an alkyl group having 1 to 20 carbon atoms, a cycloalkyl group, or an aryl group; $R^{10}$ represents a single bond or an alkylene group having 1 to 9 carbon atoms; and $R^{12}$ represents an alkylene group having 1 to 9 carbon atoms.

In a case where the compound represented by Formula (31) is used as the compound (Y1), a compound in which $R^1$ in Formula (1) represents $CH_2R^7$, $R^2$ represents $R^8$, and $R^3$ represents $R^9$ is obtained as the compound (A).

In Formula (31), the alkyl group having 1 to 9 carbon atoms as $R^7$ has the same definition as that for the alkyl group having 1 to 10 carbon atoms as $R^1$ except that the number of carbon atoms thereof is 9 or less.

$R^8$ and $R^9$ each has the same definition as that for $R^2$ and $R^3$ in Formula (1).

Examples of the compound represented by Formula (31) include 1-alkenyl alkyl ether in which X in Formula (31) represents -O-, 1-alkenyl alkyl sulfide in which X in Formula (31) represents -S-, and 1-alkenyl dialkylamine in which X in Formula (31) represents $-NR^{14}-$. Examples of the 1-alkenyl alkyl ether include vinyl ethers such as alkyl vinyl ether (such as ethyl vinyl ether, butyl vinyl ether, isobutyl vinyl ether, t-butyl vinyl ether, or 2-ethyl hexyl vinyl ether) and cycloalkyl vinyl ether (such as cyclohexyl vinyl ether); 1-propenyl ethers such as ethyl-1-propenyl ether; and 1-butenyl ethers such as ethyl-1-butenyl ether. Examples of the 1-alkenyl alkyl sulfide include 1-alkenyl alkyl sulfides such as 1-(ethenylthio) ethane, 1-(ethenylthio)propane, 1-(ethenylthio)butane, 2-(ethenylthio)butane, 1-(ethenylthio)-2-methylpropane, 1-(propylthio)-1-propene, and 2-(propylthio)-1-propene. Examples of the 1-alkenyl dialkylamine include 1-alkenyl dialkylamines such as N,N-dimethyl ethenamine, N-methyl-N-ethyl ethenamine, N,N-diethyl ethenamine, and N-vinylpyrrolidine.

Among these, 1-alkenyl alkyl ether is preferable, and vinyl ethers and 1-propenyl ethers are more preferable.

In a case where the compound represented by Formula (32) is used as the compound (Y1), a compound in which $R^4$ in Formula (1) represents $CH_2-R^{10}$ is obtained as the compound (A).

In Formula (32), the alkylene group having 1 to 9 carbon atoms as $R^{10}$ has the same definition as that for the alkylene group as $R^4$ except that the number of carbon atoms thereof is 9 or less.

Examples of the compound represented by Formula (32) include dihydrofurans such as 2,3-dihydrofuran, and 5-methyl-2,3-dihydrofuran; dihydropyrans such as 3,4-dihydro-2H-pyran and 5,6-dihydro-4-methoxy-2H-pyran; dihydrothiophenes such as 2,3-dihydrothiophene; dihydrothiopyrans such as 3,4-dihydro-2H-thiopyran; dihydropyrroles such as 2,3-dihydro-1-methylpyrrole; and tetrahydropyridines such as 1,2,3,4-tetrahydro-1-methylpyridine.

Among these, dihydrofurans and dihydropyrans are preferable, and dihydropyrans are more preferable.

In a case where a compound represented by Formula (33) is used as the compound (Y1), a compound in which $R^5$ in Formula (1) represents $R^{11}$ and $R^6$ represents $CH_2-R^{12}$ is obtained.

In Formula (33), $R^{11}$ has the same definition as that for $R^5$. $R^{12}$ has the same definition as that for $R^6$ except that the number of carbon atoms thereof is 9 or less.

Examples of the compound represented by Formula (33) include 1-alkoxy-1-cycloalkylenes such as 1-methoxy-1-cyclopentene, 1-methoxy-1-cyclohexene, 1-methoxy-1-cycloheptene, 1-ethoxy-1-cyclopentene, 1-ethoxy-1-cyclohexene, 1-butoxy-1-cyclopentene, and 1-butoxy-1-cyclohexene; substituent-containing 1-alkoxy-1-cycloalkylenes such as 1-ethoxy-3-methyl-1-cyclohexene; 1-(alkylthio)-1-cycloalkylenes such as 1-(methylthio)-1-cyclopentene and 1-(methylthio)-1-cyclohexene; and 1-(1-pyrrolidinyl)-1-cycloalkylenes such as 1-(1-pyrrolidinyl)-1-cyclopentene and 1-(1-pyrrolidinyl)-1-cyclohexene.

The compound (A) can be obtained by reacting the compound (A0) with the compound (Y1) while maintaining the reaction temperature at 40°C to 150°C for 5 to 30 hours in the presence or absence of an acidic catalyst such as hydrochloric acid, sulfuric acid, or phosphoric acid.

[Vinyl-based copolymer (B)]

The vinyl-based copolymer (B) is a copolymer polymerized from a monomer having a vinyl group, and examples thereof include an acrylic copolymer, a vinyl chloride copolymer, a styrene copolymer, a vinyl acetate copolymer, and a vinyl ether copolymer. Examples thereof further include chlorinated polyolefins such as chlorinated polyethylene and

chlorinated polypropylene; polyvinyl ether; vinyl chloride copolymer such as polyvinyl acetate, vinyl chloride-vinyl acetate copolymer, vinyl chloride-vinyl propionate copolymer, vinyl chloride-isobutyl vinyl ether copolymer, vinyl chloride-isopropyl vinyl ether copolymer, vinyl chloride-ethyl vinyl ether copolymer; chlorinated paraffin; chlorinated rubber; and polyether polyol, and (meth)acrylic acid ester copolymer.

The vinyl-based copolymer (B) is preferably a (meth)acrylic copolymer including at least one constituent unit selected from the group consisting of a constituent unit (hereinafter, also referred to as a "constituent unit (u1)") having at least one structure (I) represented by Formula (4), Formula (5), or Formula (6), a constituent unit (hereinafter, also referred to as a "constituent unit (u2)") containing a triorganosilyloxycarbonyl group, and a constituent unit (hereinafter, also referred to as a "constituent unit (u3)") having at least one structure (III) represented by Formula (7) or Formula (8).

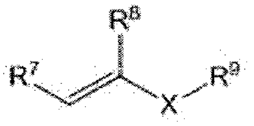
(3 1)

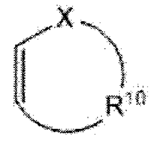
(3 2)

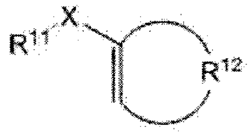
(3 3)

In each of the formulae, among single lines extending from carbon atoms of a carbonyl group, the line that is not bonded to an oxygen atom refers to a bonding site.

In Formula (4), $R^{15}$, $R^{16}$, and $R^{17}$ each has the same definition as that for $R^1$, $R^2$, and $R^3$ in Formula (1), and the preferable aspects thereof are also the same as described above. In Formula (5), $R^{18}$ has the same definition as that for $R^4$ in Formula (2), and the preferable aspects thereof are the same as described above. In Formula (6), $R^{19}$ and $R^{20}$ each has the same definition as that for $R^5$ and $R^6$ in Formula (3). In Formulae (4) to (6), X and $-NR^{21}$-has the same definition as that for $-NR^{14}$ in Formulae (1) to (3), and the preferable aspects thereof are the same as described above.

-COO-M-OCO ··· (7)

$-COO-M-R^{13}$ ··· (8)

In the formulae, M represents Zn, Cu, Mg, or Ca, and $R^{13}$ represents an organic acid residue other than a (meth) acryloyloxy group.

The vinyl-based copolymer (B) may further have a constituent unit (hereinafter, also referred to as a "constituent unit (u4)") other than the constituent unit (u1), the constituent unit (u2), and the constituent unit (u3).

(Constituent unit (u1))

The constituent unit (u1) has a structure in which an ethylenically unsaturated bond of a monomer (m1) is cleaved to form a single bond.

From the viewpoint that the viscosity at the time of dissolution of the vinyl-based copolymer (B) in a solvent becomes low, it is preferable that the monomer (m1) be a monofunctional monomer having one ethylenically unsaturated bond.

Examples of the monomer (m1) include a compound represented by Formula (11), a compound represented by Formula (12), and a compound represented by Formula (13).

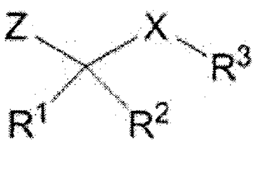
(1 1)

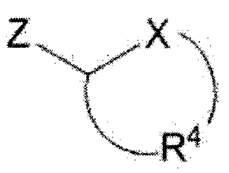
(1 2)

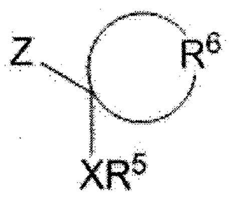
(1 3)

In the formulae, Z represents $CH_2$=CH-COO-, $CH_2=C(CH_3)$-COO-, $CHR^X$=CH-COO-, $CH_2=C(CH_2R^X)$-COO-, or $CH_2=CR^X-CH_2COO-$, $R^X$ represents the structure (I) shown above or an alkyl ester group, X represents -O-, -S-, or $-NR^{21}$-, where $R^{21}$ represents a hydrogen atom or an alkyl group, and $R^1$ to $R^6$ each has the same definition as described above.

$CH_2$=CH-COO- as Z represents an acryloyloxy group, and $CH_2$=C($CH_3$)-COO-represents a methacryloyloxy group.

CH($CH_3$)=CH-COO- represents a crotonoyloxy group (an ethylenically unsaturated bond is of a trans type) or an isocrotonoyloxy group (an ethylenically unsaturated bond is of a cis type).

$CHR^X$=CH-COO- represents a maleinoyloxy group (an ethylenically unsaturated bond is of a cis type) or a fumaroyloxy group (an ethylenically unsaturated bond is of a trans type), in which a carboxy group is substituted with a structure (I) or an alkyl ester group.

The structure (I) in $R^X$ has the same definition as described above. It is preferable that $R^X$ have the same structure as that of the group to which Z is bonded. For example, in a case of the compound represented by Formula (11), it is preferable that $R^X$ represent a group represented by $-CR^1R^2-OR^3$.

The alkyl ester group as $R^X$ is represented by $-COOR^{X1}$. $R^{X1}$ represents an alkyl group. As the alkyl group represented by $R^{X1}$, an alkyl group having 1 to 6 carbon atoms is preferable, and a methyl group is particularly preferable.

$CH_2$=C($CH_2R^X$)-COO- or $CH_2$=$CR^X$-$CH_2$COO- represents an itaconoyloxy group in which a carboxy group is substituted with a structure (I) or an alkyl ester group. $R^X$ has the same definition as described above.

It is preferable that Z represent $CH_2$=CH-COO- or CH($CH_3$)=CH-COO-.

Examples of the monomer (m1) are the same as those described below.

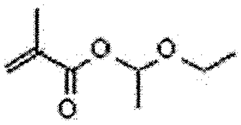 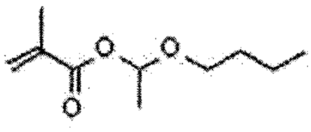 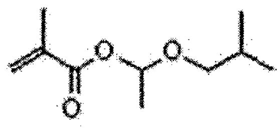

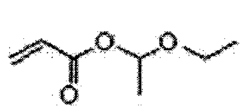 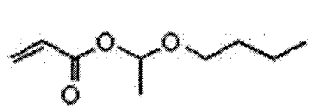 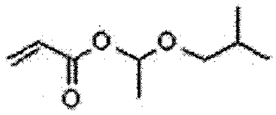

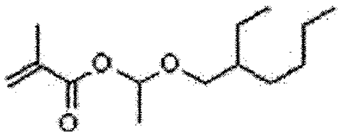 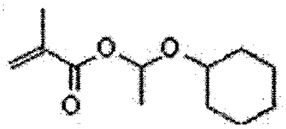 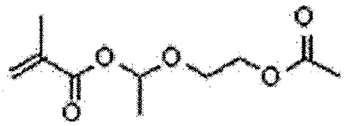

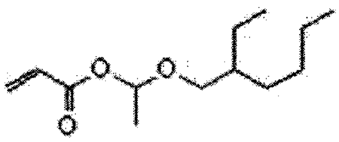 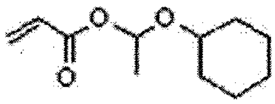 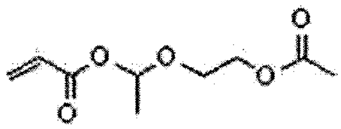

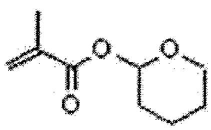 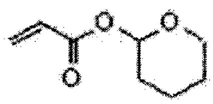 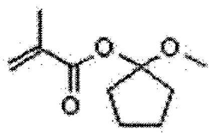 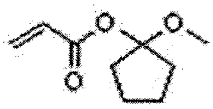

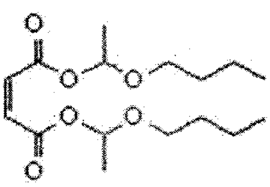 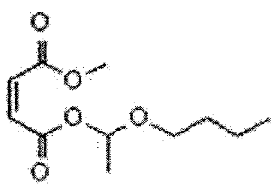

(Constituent unit (u2))

The constituent unit (u2) contains a triorganosilyloxycarbonyl group. Examples of the triorganosilyloxycarbonyl group include a group represented by Formula (II).

$$-COO-SiR^{41}R^{42}R^{43} \cdots \quad (II)$$

In Formula (II), $R^{41}$ to $R^{43}$ each represents a hydrocarbon group having 1 to 20 carbon atoms.

Examples of the hydrocarbon group as $R^{41}$ to $R^{43}$ include an alkyl group having 1 to 20 carbon atoms such as a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, or a tetradecyl group; a cycloalkyl group such as a cyclohexyl group; and an aryl group such as a phenyl group or a naphthyl group.

Each of the cycloalkyl group and the aryl group may have a substituent. Examples of the substituent include a halogen atom, an alkyl group, an acyl group, a nitro group, and an amino group. The number of carbon atoms in the alkyl group as a substituent is preferably in a range of 1 to 18.

$R^{41}$ to $R^{43}$ may be the same as or different from one another.

From the viewpoints of obtaining a coating film exhibiting a stabilized polishing rate and maintaining the stabilized antifouling performance for a long period of time, it is preferable that at least one of $R^{41}$ to $R^{43}$ represent an isopropyl group and particularly preferable that all of $R^{41}$ to $R^{43}$ represent an isopropyl group.

The constituent unit (u2) is typically a monomer (m2) containing a triorganosilyloxycarbonyl group.

The constituent unit (u2) has a structure in which an ethylenically unsaturated bond of a monomer (m2) is cleaved to form a single bond.

From the viewpoint that the viscosity at the time of dissolution of the vinyl-based copolymer (B) in a solvent becomes low, it is preferable that the monomer (m2) be a monofunctional monomer having one ethylenically unsaturated bond.

Examples of the monomer (m2) include a monomer represented by Formula (m2-1) and a monomer represented by Formula (m2-2). Among these, a monomer represented by Formula (m2-1) is preferable.

$$CH_2=C(R^{44})-COO-SiR^{41}R^{42}R^{41} \cdots \quad (m2-1)$$

$$CH(COOR^{45})=C(R^{44})-COO-SiR^{41}R^{42}R^{43} \cdots \quad (m2-2)$$

(In the formulae, $R^{41}$ to $R^{43}$ each has the same definition as described above, $R^{44}$ represents a hydrogen atom or a methyl group, and $R^{45}$ represents an alkyl group.)

Specific examples of the monomer represented by Formula (m2-1) are the same as those described below. Specific examples of the monomer represented by Formula (m2-1) include trimethylsilyl (meth)acrylate, triethylsilyl (meth)acrylate, tri-n-propylsilyl (meth)acrylate, tri-n-butylsilyl (meth)acrylate, tri-n-amylsilyl (meth)acrylate, tri-n-hexylsilyl (meth)acrylate, tri-n-octylsilyl (meth)acrylate, tri-n-dodecylsilyl (meth)acrylate, triphenylsilyl (meth)acrylate, tri-p-methylphenylsilyl (meth)acrylate, tribenzylsilyl (meth)acrylate, triisopropylsilyl (meth)acrylate, triisobutylsilyl (meth)acrylate, tri-s-butylsilyl (meth)acrylate, tri-2-methylisopropylsilyl (meth)acrylate, tri-t-butylsilyl (meth)acrylate, ethyldimethylsilyl (meth)acrylate, n-butyldimethylsilyl (meth)acrylate, diisopropyl-n-butylsilyl (meth)acrylate, n-octyl di-n-butylsilyl (meth)acrylate, diisopropylstearylsilyl (meth)acrylate, dicyclohexylphenylsilyl (meth)acrylate, t-butyldiphenylsilyl (meth)acrylate, and lauryldiphenylsilyl (meth)acrylate.

In Formula (m2-2), examples of the alkyl group as $R^{45}$ include an alkyl group having 1 to 5 carbon atoms.

Specific examples of the compound represented by Formula (m2-2) are the same as those described below. Specific examples of the compound represented by Formula (m2-2) include triisopropylsilyl methyl malate, triisopropylsilyl amyl malate, tri-n-butylsilyl-n-butyl malate, t-butyldiphenylsilyl methyl malate, t-butyldiphenylsilyl-n-butyl malate, triisopropylsilyl methyl fumarate, triisopropylsilyl amyl fumarate, tri-n-butylsilyl-n-butyl fumarate, t-butyldiphenylsilyl methyl fumarate, and t-butyldiphenylsilyl-n-butyl fumarate.

(Constituent unit (u3))

The constituent unit (u3) has a structure in which an ethylenically unsaturated bond of a monomer (m3) is cleaved to form a single bond.

The constituent unit (u3) has at least one structure (III) selected from the group consisting of a structure represented by Formula (7) or (8).

$$-COO-M-OCO \cdots \quad (7)$$

$$-COO-M-R^{13} \cdots \quad (8)$$

(In the formulae, M represents Zn, Cu, Mg, or Ca; and $R^{13}$ represents an organic acid residue other than a (meth)acryloyloxy group.)

It is preferable that M represent Zn or Cu.

The organic acid residue in $R^{13}$ refers to a remaining part obtained by removing one proton from an organic acid (for example, a remaining part obtained by removing a proton from a carboxy group of a carboxylic acid) and is ion-bonded to M in place of this proton.

As the organic acid, a carboxylic acid is preferable, and examples thereof include a monocarboxylic acid such as monochloroacetic acid, monofluoroacetic acid, acetic acid, propionic acid, octylic acid, versatic acid, isostearic acid, palmitic acid, cresotic acid, $\alpha$-naphthoic acid, $\beta$-naphthoic acid, benzoic acid, 2,4,5-trichlorophenoxyaceitc acid, 2,4-dichlorophenoxyacetic acid, quinolinecarboxylic acid, nitrobenzoic acid, nitronaphthalenecarboxylic acid, pyruvic acid, naphthenic acid, abietic acid, or hydrogenated abietic acid.

From the viewpoint of obtaining a coating film with high durability which can prevent cracking or peeling for a long period of time, it is preferable that $R^{13}$ represent a fatty acid residue (aliphatic monocarboxylic acid residue) having 1 to 20 carbon atoms.

The constituent unit (u3) is a monomer (m3) having the structure (III).

Examples of the monomer (m3) having the structure (III) include a monomer in which a vinyl group having an unsubstituted or substituted group is bonded to both terminals of a group represented by Formula (7) and a monomer in which a vinyl group having an unsubstituted or substituted group is bonded to one terminal (a side opposite to the $R^{13}$ side) of a group represented by Formula (8).

Examples of the monomer in which the vinyl group is bonded to both terminals of the group represented by Formula (7) include a monomer (hereinafter, also referred to as a "monomer (m3-1)") represented by Formula (m3-1).

Examples of the monomer in which the vinyl group is bonded to one terminal of the group represented by Formula (8) include a monomer (hereinafter, also referred to as a "monomer (m3-2)") represented by Formula (m3-2).

$$(CH_2=C(R^{31})\text{-}COO)_2M \cdots \qquad (m3\text{-}1)$$

$$CH_2=C(R^{31})\text{-}COO\text{-}M\text{-}R^{32} \cdots \qquad (m3\text{-}2)$$

In the formulae, M represents Zn, Cu, Mg, or Ca, $R^{31}$ represents a hydrogen atom or a methyl group, and $R^{32}$ represents an organic acid residue other than the (meth)acryloyloxy group.

M and $R^{32}$ each has the same definition as described above, and the preferable aspects thereof are the same as described above.

Examples of the monomer (m3-1) include zinc acrylate [$(CH_2=CHCOO)_2Zn$], zinc methacrylate [$(CH_2=C(CH_3)COO)_2Zn$], copper acrylate [$(CH_2=CHCOO)_2Cu$], copper methacrylate [$(CH_2=C(CH_3)COO)_2Cu$], magnesium acrylate [$(CH_2=CHCOO)_2Mg$], magnesium methacrylate [$(CH_2=C(CH_3)COO)_2Mg$], calcium acrylate [$(CH_2=CHCOO)_2Ca$], and calcium methacrylate [$(CH_2=C(CH_3)COO)_2Ca$]. These may be used alone or in combination of two or more kinds thereof.

Among these, from the viewpoint that the transparency of the vinyl-based copolymer (B) is improved and the color tone of the coating film containing the vinyl-based copolymer (B) tends to be appealing, zinc (meth)acrylate or copper (meth)acrylate is preferable.

Examples of the monomer (m3-2) include magnesium monochloroacetate (meth)acrylate, calcium monochloroacetate (meth)acrylate, zinc monochloroacetate (meth)acrylate, and copper monochloroacetate (meth)acrylate; magnesium monofluoroacetate (meth)acrylate, calcium monofluoroacetate (meth)acrylate, zinc monofluoroacetate (meth) acrylate, and copper monofluoroacetate (meth)acrylate; magnesium acetate (meth)acrylate, calcium acetate (meth) acrylate, zinc acetate (meth)acrylate, and copper acetate (meth)acrylate; magnesium propionate (meth)acrylate, calcium propionate (meth)acrylate, zinc propionate (meth)acrylate, and copper propionate (meth)acrylate; magnesium octylate (meth)acrylate, calcium octylate (meth)acrylate, zinc octylate (meth)acrylate, and copper octylate (meth)acrylate; magnesium versatate (meth)acrylate, calcium versatate (meth)acrylate, zinc versatate (meth)acrylate, and copper versatate (meth)acrylate; magnesium isostearate (meth)acrylate, calcium isostearate (meth)acrylate, zinc isostearate (meth)acrylate, and copper isostearate (meth)acrylate; magnesium palmitate (meth)acrylate, calcium palmitate (meth) acrylate, zinc palmitate (meth)acrylate, and copper palmitate (meth)acrylate; magnesium cresotinate (meth)acrylate, calcium cresotinate (meth)acrylate, zinc cresotinate (meth)acrylate, and copper cresotinate (meth)acrylate; magnesium $\alpha$-naphthoate (meth)acrylate, calcium $\alpha$-naphthoate (meth)acrylate, zinc $\alpha$-naphthoate (meth)acrylate, and copper $\alpha$-naphthoate (meth)acrylate; magnesium $\beta$-naphthoate (meth)acrylate, calcium $\beta$-naphthoate (meth)acrylate, zinc $\beta$-naphthoate (meth)acrylate, and copper $\beta$-naphthoate (meth)acrylate; magnesium benzoate (meth)acrylate, calcium benzoate (meth)acrylate, zinc benzoate (meth)acrylate, and copper benzoate (meth)acrylate; magnesium 2,4,5-trichlorophenoxy acetate (meth)acrylate, calcium 2,4,5-trichlorophenoxy acetate (meth)acrylate, zinc 2,4,5-trichlorophenoxy acetate (meth)acrylate, and copper 2,4,5-trichlorophenoxy acetate (meth)acrylate; magnesium 2,4-dichlorophenoxy acetate (meth)acrylate, calcium 2,4-dichlorophenoxy acetate (meth)acrylate, zinc 2,4-dichlorophenoxy acetate (meth) acrylate, and copper 2,4-dichlorophenoxy acetate (meth)acrylate; magnesium quinoline carboxylate (meth)acrylate,

calcium quinoline carboxylate (meth)acrylate, zinc quinoline carboxylate (meth)acrylate, and copper quinoline carboxylate (meth)acrylate; magnesium nitrobenzoate (meth)acrylate, calcium nitrobenzoate (meth)acrylate, zinc nitrobenzoate (meth)acrylate, and copper nitrobenzoate (meth)acrylate; magnesium nitronaphthalene carboxylate (meth)acrylate, calcium nitronaphthalene carboxylate (meth)acrylate, zinc nitronaphthalene carboxylate (meth)acrylate, and copper nitronaphthalene carboxylate (meth)acrylate; and magnesium pyruvate (meth)acrylate, calcium pyruvate (meth)acrylate, zinc pyruvate (meth)acrylate, and copper pyruvate (meth)acrylate. These may be used alone or in combination of two or more kinds thereof.

Among these, from the viewpoint that the transparency of a copolymer (A-2) is improved and the color tone of the coating film containing the copolymer (A-2) tends to be appealing, a zinc-containing monomer in which M represents Zn is preferable. Further, from the viewpoint of the durability of the coating film to be obtained, fatty acid zinc (meth)acrylate (in which M in Formula (m3-2) represents Zn and R32 represents a fatty acid residue) or fatty acid copper (meth)acrylate (in which M in Formula (m3-2) represents Cu and R32 represents a fatty acid residue) is more preferable.

From the viewpoints of maintaining the self-polishing property of the coating film to be obtained for a long period of time and obtaining an excellent antifouling property, it is preferable that the monomer (m3) contain both of the monomer (m3-1) and the monomer (m3-2).

As the combination of the monomer (m3-1) and the monomer (m3-2), a combination of zinc (meth)acrylate and fatty acid zinc (meth)acrylate or copper (meth)acrylate and fatty acid copper (meth)acrylate is preferable.

In a case where the vinyl-based copolymer (B) has both of the monomer (m3-1) unit and the monomer (m, 3-2) unit, the ratio (molar ratio) (monomer (m3-1) unit/monomer (m3-2) unit) of the monomer (m3-1) unit to the monomer (m3-2) unit in the vinyl-based copolymer (B) is preferably in a range of 10/90 to 90/10, more preferably in a range of 20/80 to 80/20, and still more preferably in a range of 30/70 to 70/30. In a case where the ratio thereof is 90/10 or less, the crack resistance or the adhesiveness of the coating film is improved. In a case where the ratio thereof is 10/90 or greater, the viscosity of the paint tends to be lowered.

The monomer (m3-1) is obtained according to a method of reacting an inorganic metal compound having a metal element corresponding to M in Formula (m3-1) and (meth)acrylic acid in a diluent such as an organic solvent or a reactive diluent containing a polymerizable unsaturated group such as an ethylenically unsaturated monomer. The mixture containing a metal-containing polymerizable monomer obtained using this method has an excellent compatibility with an organic solvent or another monomer so that the polymerization can be easily carried out. It is preferable that the reaction be performed in the presence of water, and the content of water in the reactant preferably be in a range of 0.01% to 30% by mass. Examples of the inorganic metal compound include an oxide, a hydroxide, and a chloride of a metal selected from Zn, Cu, Mg, and Ca.

The monomer (m3-2) is obtained according to a method of reacting an inorganic metal compound having a metal element corresponding to M in Formula (m3-2), (meth)acrylic acid, and an organic acid corresponding to $R^{12}$ as an organic acid residue in Formula (32) in a diluent such as an organic solvent or a reactive diluent containing a polymerizable unsaturated group such as an ethylenically unsaturated monomer. Examples of the inorganic metal compound are the same as those exemplified as the inorganic metal compound used for obtaining the monomer (m3-1).

The monomer mixture containing the monomer (m3-1) and the monomer (m3-2) is obtained according to a method of reacting an inorganic metal compound having a metal element corresponding to M in Formulae (m3-1) and (m3-2), (meth)acrylic acid, and an organic acid corresponding to R32 as an organic acid residue in Formula (m3-2) in a diluent such as an organic solvent or a reactive diluent such as an ethylenically unsaturated monomer.

The amount of the organic acid to be used, which corresponds to $R^{32}$, is preferably in a range of 0.01 to 3 mol times, more preferably in a range of 0.01 to 0.95 mol times, and still more preferably in a range of 0.1 to 0.7 mol times with respect to the amount of the inorganic metal compound. In a case where the content of the organic acid is 0.01 mol times or greater, precipitation of a solid in a step of producing this monomer mixture is suppressed, and the self-polishing property and crack resistance of a coating film to be obtained are improved. In a case where the content thereof is 3 mol times or less, the antifouling property of a coating film to be obtained tends to be maintained for a long period of time.

(Constituent unit (u4))

The constituent unit (u4) is a constituent unit other than the constituent unit (u1), the constituent unit (u2), and the constituent unit (u3). The constituent unit (u4) has a structure in which an ethylenically unsaturated bond of a monomer (m4) that contains an ethylenically unsaturated bond and does not contain the structure (I), the triorganosilyloxycarbonyl group, and the structure (III) is cleaved to form a single bond.

Examples of the monomer (m4) are the same as those described below.

Examples of the monomer (m4) include hydrophobic group-containing (meth)acrylic acid ester monomers such as substituted or unsubstituted alkyl (meth)acrylates [for example, methyl (meth)acrylate, ethyl (meth)acrylate, n-propyl (meth)acrylate, i-propyl (meth)acrylate, n-butyl (meth)acrylate, i-butyl (meth)acrylate, t-butyl (meth)acrylate, 2-ethylhexyl (meth)acrylate, lauryl (meth)acrylate, stearyl (meth)acrylate, behenyl (meth)acrylate, 1-methyl-2-methoxyethyl (meth)acrylate, 3-methoxybutyl (meth)acrylate, and 3-methyl-3-methoxybutyl (meth)acrylate], substituted or unsubstituted aralkyl (meth)acrylate [for example, benzyl (meth)acrylate, m-methoxyphenylethyl (meth)acrylate, and p-methoxyphenylethyl (meth)acrylate], substituted or unsubstituted aryl (meth)acrylates [for example, phenyl (meth)acrylate, m-methoxyphenyl (meth)acrylate, p-methoxyphenyl (meth)acrylate, and o-methoxyphenylethyl (meth)acrylate], alicyclic (meth)acrylate [for example, isobornyl (meth)acrylate, and cyclohexyl (meth)acrylate], trifluoroethyl (meth)acrylate, perfluorooctyl (meth)acrylate, and perfluorocyclohexyl (meth)acrylate;

oxyethylene group-containing (meth)acrylic acid ester monomers such as 2-methoxyethyl (meth)acrylate, 2-ethoxyethyl (meth)acrylate, 2-butoxyethyl (meth)acrylate, butoxydiethylene glycol (meth)acrylate, methoxytriethylene glycol (meth)acrylate, methoxypolyethylene glycol (meth)acrylate, phenoxyethyl (meth)acrylate, and 2- (2-ethylhexaoxy)ethyl (meth)acrylate;
hydroxy group-containing (meth)acrylic acid ester monomers such as 2-hydroxyethyl (meth)acrylate, 2-hydroxypropyl (meth)acrylate, 4-hydroxybutyl (meth)acrylate, and glycerol (meth)acrylate;
terminal alkoxyallylated polyether monomers such as methoxypolyethylene glycol allyl ether, methoxypolypropylene glycol allyl ether, butoxypolyethylene glycol allyl ether, butoxypolypropylene glycol allyl ether, methoxypolyethylene glycol-polypropylene glycol allyl ether, and butoxypolyethylene glycol-polypropylene glycol allyl ether;
epoxy group-containing vinyl monomers such as (meth)glycidyl acrylate, glycidyl $\alpha$-ethylacrylate, and (meth)acrylic acid 3,4-epoxy butyl;
primary or secondary amino group-containing vinyl monomers such as butyl aminoethyl (meth)acrylate and (meth) acrylamide;
tertiary amino group-containing vinyl monomers such as dimethylaminoethyl (meth)acrylate, diethylaminoethyl (meth)acrylate, dimethylaminopropyl (meth)acrylate, dimethylaminobutyl (meth)acrylate, dibutylaminoethyl (meth) acrylate, dimethylaminoethyl (meth)acrylamide, and dimethylaminopropyl (meth)acrylamide;
heterocyclic basic monomers such as vinylpyrrolidone, vinyl pyridine, and vinyl carbazole;
acid anhydride group-containing vinyl monomers such as maleic anhydride and itaconic anhydride;
carboxy group-containing ethylenically unsaturated monomers such as methacrylic acid, acrylic acid, crotonic acid, vinyl benzoic acid, fumaric acid, itaconic acid, maleic acid, citraconic acid, monomethyl maleate, monoethyl maleate, monobutyl maleate, monooctyl maleate, monomethyl itaconic acid, monoethyl itaconic acid, monobutyl itaconic acid, monooctyl itaconic acid, monomethyl fumarate, monoethyl fumarate, monobutyl fumarate, monooctyl fumarate, monoethyl citraconic acid, monohydroxyethyl tetrahydrophthalate (meth)acrylate, monohydroxypropyl (meth)acrylate, tetrahydrophthalate, monohydroxybutyl (meth)acrylate, tetrahydrophthalate, monohydroxyethyl phthalate (meth)acrylate, monohydroxypropyl phthalate (meth)acrylate, monohydroxyethyl succinate (meth)acrylate, monohydroxypropyl succinate (meth)acrylate, monohydroxyethyl maleate (meth)acrylate, and monohydroxypropyl maleate (meth)acrylate;
unsaturated dicarboxylic acid diester monomers such as dimethyl malate, dibutyl malate, dimethyl fumarate, dibutyl fumarate, dibutyl itaconate, and diperfluorocyclohexyl fumarate;
cyano group-containing vinyl monomers such as acrylonitrile and methacrylonitrile;
vinyl ether monomers such as alkyl vinyl ether [for example, ethyl vinyl ether, propyl vinyl ether, butyl vinyl ether, hexyl vinyl ether, and 2-ethylhexyl vinyl ether], and cycloalkyl vinyl ether [for example, cyclohexyl vinyl ether];
vinyl ester monomers such as vinyl acetate, vinyl propionate, vinyl butyrate, and vinyl benzoate;
aromatic vinyl monomers such as styrene, vinyl toluene, and $\alpha$-methylstyrene;
halogenated olefins such as vinyl chloride, vinylidene chloride, vinyl fluoride, vinylidene fluoride, and chlorotrifluoroethylene;
polyfunctional monomers such as ethylene glycol di(meth)acrylate, diethylene glycol di(meth)acrylate, triethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, 1,9-nonanediol di(meth)acrylate, 1,10-decanediol di(meth)acrylate, trimethylolpropane tri(meth)acrylate, pentaerythritol tetra (meth)acrylate, dipentaerythritol hexa(meth)acrylate, allyl methacrylate, triallyl cyanurate, diallyl maleate, and polypropylene glycol diallyl ether; and macromonomers.

One or more kinds of these can be suitably selected as necessary.
Examples of the macromonomer include a compound which has an ethylenically unsaturated bond-containing group, and two or more constituent units derived from a monomer having an ethylenically unsaturated bond-containing group. Two or more constituent units of the macromonomer may be the same as or different from each other.
Examples of the ethylenically unsaturated bond-containing group include $CH_2=C(COOR)-CH_2-$, a (meth) acryloyl group, a 2-(hydroxymethyl)acryloyl group, and a vinyl group. R represents a hydrogen atom, an unsubstituted or substituted alkyl group, an unsubstituted or substituted alicyclic group, an unsubstituted or substituted aryl group, and an unsubstituted or substituted heterocyclic group. Examples of the substituent include at least one selected from the group consisting of an alkyl group (excluding a case where R represents an alkyl group having a substituent), an aryl group,

$-COOR^{61}$, a cyano group, $-OR^{62}$, $-NR^{63}R^{64}$, $-CONR^{65}R^{66}$, a halogen atom, an allyl group, an epoxy group, a siloxy group, and a hydrophilic or ionic group. $R^{61}$ to $R^{66}$ each independently represents a hydrogen atom, an alkyl group, an alicyclic group, or an aryl group.

As the monomer having the ethylenically unsaturated bond-containing group, for example, various monomers described above as examples of the monomer (m4) can be used (excluding macromonomers), and various monomers described above as examples of the monomers (m1) to (m3) can also be used in combination.

Examples of the macromonomer include monomers disclosed in PCT International Publication No. WO 2013/108880.

From the viewpoint that the vinyl-based copolymer (B) is easily formed to have a high solid content and a low viscosity at the time of being dissolved in a solvent, it is preferable that the monomer (m4) be a monofunctional monomer having one ethylenically unsaturated bond and particularly preferable that an ethylenically unsaturated bond be derived from an acryloyl group. In other words, it is particularly preferable that the monomer (m4) be a monofunctional monomer containing one acryloyl group.

From the viewpoint of further improving the flexibility or the crack resistance and the peeling resistance of a coating film and the self-polishing property for a long period of time with a good balance therebetween, it is preferable that the constituent unit (u4) have a constituent unit derived from a hydrophobic group-containing (meth)acrylic acid ester monomer.

As the hydrophobic group-containing (meth)acrylic acid ester monomer, an alkyl (meth)acrylate is preferable.

From the viewpoint of improving the solubility or the crack resistance of a coating film, it is preferable that the constituent unit (u4) have a constituent unit derived from the oxyethylene group-containing (meth)acrylic acid ester monomer.

As the oxyethylene group-containing (meth)acrylic acid ester monomer, a compound represented by Formula (4-1) is preferable.

$$Z^1\text{-}(CH_2CH_2O)_nR^{22} \qquad (4\text{-}1)$$

(In formula, $Z^1$ represents an acryloyloxy group or a methacryloyloxy group, $R^{22}$ represents a hydrogen atom, an alkyl group having 1 to 10 carbon atoms, or an aryl group, and n represents an integer of 1 to 15.)

In Formula (4-1), as a result of comparison of a case where $Z^1$ represents an acryloyloxy group to a case where $Z^1$ represents a methacryloyloxy group, the hydrolysis rate tends to be higher in the case where $Z^1$ represents an acryloyloxy group, and any case can be optionally selected depending on the dissolution rate.

Examples of the alkyl group having 1 to 10 carbon atoms, and the aryl group as $R^{22}$ are the same as those exemplified as $R^1$ and $R^3$.

From the viewpoints of the water resistance and the crack resistance, n represents preferably an integer of 1 to 10, more preferably an integer of 1 to 5, still more preferably an integer of 1 to 3, and particularly preferably 1 or 2.

(Content of each constituent unit)

The content of the constituent units (u1) to (u3) in the vinyl-based copolymer (B) is preferably in a range of 1% to 99% by mass, more preferably in a range of 2% to 90% by mass, and still more preferably in a range of 5% to 70% by mass with respect to the total amount (100% by mass) of all constituent units. In a case where the content of each of the constituent units (u1) to (u3) is greater than or equal to the lower limit of the above-described range, the self-polishing property of a coating film is further improved. In a case where the content of each of the constituent units (u1) to (u3) is less than or equal to the upper limit of the above-described range, the coating film has suitable hydrolyzability, the self-polishing property is maintained for a long period of time, and the antifouling effect is further improved.

The content of the constituent unit (u4) is preferably in a range of 1% to 99% by mass, more preferably in a range of 10% to 98% by mass, and still more preferably in a range of 30% to 95% by mass with respect to the total amount (100% by mass) of all constituent units.

It is preferable that the constituent unit of the vinyl-based copolymer (B) contain a constituent unit derived from a (meth)acrylic monomer. The proportion of the constituent units derived from the (meth)acrylic monomer with respect to the total amount (100% by mass) of all constituent units in the vinyl-based copolymer (B) is preferably in a range of 20% to 100% by mass and more preferably in a range of 40% to 100% by mass.

In a case where the vinyl-based copolymer (B) has a constituent unit derived from the oxyethylene group-containing (meth)acrylic acid ester monomer, the content of the constituent unit is preferably in a range of 1% to 80% by mass, and more preferably 5% to 40% by mass, with respect to the total amount of all constituent units. In a case where the content of the constituent unit is greater than or equal to the lower limit of the above-described range, hydrophilicity and the self-polishing property of a coating film are further improved. In a case where the content of the constituent unit is less than or equal to the upper limit of the above-described range, the coating film has suitable hydrolyzability, the self-polishing

property is maintained for a long period of time, and the antifouling effect is further improved.

In a case where the vinyl-based copolymer (B) has a constituent unit derived from the monomer (m4) other than the constituent unit derived from the oxyethylene group-containing (meth)acrylic acid ester monomer, such as a hydrophobic group-containing (meth)acrylic acid ester monomer, the content of the constituent unit is preferably in a range of 1% to 90% by mass, and more preferably 10% to 80% by mass, with respect to the total amount of all constituent units. In a case where the content of the constituent unit is within the above-described range, flexibility or the crack resistance and the peeling resistance, and the antifouling effect of a coating film are further improved. In a case where the content of the constituent unit is less than or equal to the upper limit of the above-described range, the coating film has suitable hydrolyzability, the self-polishing property is maintained for a long period of time, and the antifouling effect is further improved.

The total amount of the constituent unit (u1), the constituent unit (u2), the constituent unit (u3), and the constituent unit (u4) is 100% by mass.

The content (% by mass) of each constituent unit in the copolymer can be measured by a known method such as gas chromatography, high performance liquid chromatography, or nuclear magnetic resonance spectroscopy.

The weight-average molecular weight (Mw) of the vinyl-based copolymer (B) is preferably in a range of 1000 to 100000, more preferably in a range of 2000 to 80000, and still more preferably in a range of 3000 to 60000.

In a case where the weight-average molecular weight of the vinyl-based copolymer (B) is less than or equal to the upper limit of the above-described range, the viscosity of the solution at the time of dissolving the vinyl-based copolymer (B) in a solvent becomes lower, and a composition with a high solid content and a low viscosity is easily obtained as the antifouling paint composition. Further, the antifouling property of a coating film is improved. The weight-average molecular weight thereof is greater than or equal to the lower limit of the above-described range, the hardness and the durability of a coating film are further improved.

The number-average molecular weight (Mn) of the vinyl-based copolymer (B) is preferably in a range of 500 to 50000 and more preferably in a range of 1000 to 40000.

The molecular weight distribution (Mw/Mn) of the vinyl-based copolymer (B) is preferably in a range of 1.5 to 5.0 and more preferably in a range of 2.2 to 3.0.

The weight-average molecular weight and the number-average molecular weight of the vinyl-based copolymer (B) are measured by gel permeation chromatography (GPC) using polystyrene as a reference resin.

(Method of producing vinyl-based copolymer (B))

As a method for producing the vinyl-based copolymer (B), for example, known polymerization methods such as a solution polymerization method, a suspension polymerization method, a massive polymerization method, and an emulsion polymerization method are applicable. From the viewpoints of the productivity and the performance of the coating film, a solution polymerization method is preferable.

The polymerization may be performed according to a known method using a known polymerization initiator. Examples of known methods include a method of allowing the monomer mixture to react at a reaction temperature of 60°C to 120°C for 4 to 14 hours in the presence of a radical initiator. During the polymerization, a chain transfer agent may be used as necessary.

As the radical initiator, a known initiator can be used, and examples thereof include 2,2'-azobisisobutyronitrile, 2,2'-azobis(2,4-dimethyl valeronitrile), or 2,2'-azobis(2-methylbutyronitrile), benzoyl peroxide, cumene hydroperoxide, lauryl peroxide, di-t-butyl peroxide, and t-butylperoxy-2-ethylhexanoate.

The content of the polymerization initiator is not particularly limited and can be suitably set. Typically, the content of the polymerization initiator is approximately in a range of 0.1 to 20 parts by mass with respect to 100 parts by mass of the polymerizable monomer.

As the chain transfer agent, a known agent can be used, and examples thereof include mercaptans such as n-dodecyl mercaptan, thioglycolic acid esters such as octyl thioglycolate, an α-methylstyrene dimer, and terpinolene.

The content of the chain transfer agent is not particularly limited and can be suitably set. Typically, the content of the chain transfer agent is approximately in a range of 0.0001 to 10 parts by mass with respect to 100 parts by mass of the polymerizable monomer.

As the solvent used for the solution polymerization, for example, a typical organic solvent such as toluene, xylene, methyl isobutyl ketone, or n-butyl acetate can be used.

Examples of the method of producing the vinyl-based copolymer (B) having the structure (I) include the following production methods (α) and (β).

Production method (α): Method of polymerizing a monomer mixture containing the monomer (m1)

Production method (β): Method of polymerizing a monomer mixture containing a monomer (m0) having an ethylenically unsaturated bond and a carboxy group to obtain a copolymer (B0) having a carboxy group, and converting the

carboxy group of the copolymer (B0) into the structure (I)

"Production method (α)"

Monomer mixture:

The monomer mixture used in the production method (α) contains a monomer of the monomer (m1), and may further contain the monomers (m2) to (m4).

As the monomers (m1) to (m4), a commercially available products may be used respectively, or the monomers can be suitably synthesized according to a known method.

The monomer (m1) can be synthesized by converting a carboxy group in the monomer (m0) having the ethylenically unsaturated bond and the carboxy group to the structure (I).

Examples of the monomer (m0) include (meth)acrylic acid, crotonic acid, isocrotonic acid, maleic acid, fumaric acid, itaconic acid, monomethyl maleate, and monomethyl fumarate.

Examples of the method of converting the carboxy group in the monomer (m0) to the structure (I) include a method of causing a reaction (addition reaction) between the monomer (m0) and a compound (Y2). Examples of the compound (Y2) are the same as those exemplified as those of the compound (Y1).

In a case where a compound represented by Formula (31) is used as the compound (Y2), a compound in which $R^1$ in Formula (11) represents $CH_2R^7$, $R^2$ represents $R^8$, and $R^3$ represents $R^9$ is obtained as the monomer (m1).

In Formula (31), the alkyl group having 1 to 9 carbon atoms as $R^7$ has the same definition as that for the alkyl group having 1 to 10 carbon atoms as $R^1$ except that the number of carbon atoms thereof is 9 or less.

$R^8$ and $R^9$ each has the same definition as that for $R^2$ and $R^3$ in Formula (11).

In a case where a compound represented by Formula (32) is used as the compound (Y2), a compound in which $R^4$ in Formula (12) represents $CH_2$-$R^{10}$ is obtained as the monomer (m1).

In Formula (32), the alkylene group having 1 to 9 carbon atoms as $R^{10}$ has the same definition as that for the alkylene group as $R^4$ except that the number of carbon atoms thereof is 9 or less.

In a case where a compound represented by Formula (33) is used as the compound (Y2), a compound in which $R^5$ in Formula (13) represents $R^{11}$ and $R^6$ represents $CH_2$-$R^{12}$ is obtained as the monomer (m1).

In Formula (33), $R^{11}$ has the same definition as that for $R^5$. $R^{12}$ has the same definition as that for $R^6$ except that the number of carbon atoms thereof is 9 or less.

The monomer (m1) can be obtained by reacting the monomer (m0) and the compound (Y2) while maintaining the reaction temperature at 40°C to 150°C for 5 to 30 hours. An acidic catalyst such as hydrochloric acid, sulfuric acid, or phosphoric acid may be used. In addition, after completion of the reaction, the target monomer can be recovered by carrying out vacuum distillation under predetermined conditions, as necessary.

"Production method (β)"

In the production method (β), first, the monomer mixture containing the monomer (m0) is polymerized to obtain the copolymer (B0) having a carboxy group. The monomer mixture may further contain the monomers (m1) to (m4).

The monomers (m0) to (m4) have the same definition as described above.

The preferable range of the monomer (m0) in the monomer mixture is the same as the preferable range of the content of the monomer (m1) in the monomer mixture in the production method (α). The preferable range of the content of the oxyethylene group-containing (meth)acrylic acid ester monomer or other monomer (m4) is the same as described above.

The polymerization of the monomer mixture can be carried out in the same manner as in the production method (α).

Next, the vinyl-based copolymer (B) is obtained by converting the carboxy group of the copolymer (B0) into the structure (I).

Examples of the method of converting the carboxy group in the copolymer (B0) to the structure (I) include a method of causing a reaction (addition reaction) between the copolymer (B0) and the compound (Y2).

The reaction between the copolymer (B0) and the compound (Y2) can be carried out in the same manner as the reaction between the monomer (m0) and the compound (Y2).

Examples of the method of producing the vinyl-based copolymer (B) having the structure (III) include a method of producing the copolymer vinyl-based copolymer (B) by the following production method (γ) or (δ), and adding an organic solvent as necessary. Among these, from the viewpoint of water resistance, a method of producing the vinyl-based copolymer (B) by the production method (γ), and adding an organic solvent as necessary is preferable.

Production method (γ): Method of polymerizing a monomer mixture containing the monomer (m3)

Production method (δ): Method of polymerizing a monomer mixture (δ1) containing the monomer (m0) having an ethylenically unsaturated bond and a carboxy group to obtain a copolymer (B0') having a carboxy group, and converting the carboxy group of the copolymer (B0') into the structure (III)

Examples of the method of converting the carboxy group of the copolymer (B0') into the structure (III) include a method of reacting the copolymer (B0') with an organic acid metal salt such as copper acetate or zinc acetate. The metal of the organic acid metal salt corresponds to M described above.

The reaction between the copolymer (B0') and the organic acid metal salt can be carried out by increasing the temperature to the reflux temperature and continuing the reaction for 10 to 20 hours while removing the mixed solution of an organic acid such as acetic acid to be distilled, water, and an organic solvent and replenishing the same amount of the organic solvent.

The vinyl-based copolymer (B) has at least one of the structure (I) in which the carboxy group is protected by a specific group, a triorganosilyloxycarbonyl group, and the structure (III), and is thus hydrolyzable in seawater or the like. Therefore, the coating film containing the vinyl-based copolymer (B) exhibits a self-polishing property in sea water or the like. That is, the vinyl-based copolymer (B) is protected by a specific group, and thus, does not dissolve in seawater in this state. However, in a case where the specific group is hydrolyzed by contact with seawater, a carboxy group or the like is generated, and the coating film is dissolved in seawater. The surface of the coating film is gradually dissolved in sea water, and thus the surface thereof is renewed (self-polished).

In addition, the vinyl-based copolymer (B) can be in a form of a solution having a high solid content and a low viscosity when an organic solvent is added. In a case where a resin composition containing the vinyl-based copolymer (B) and the organic solvent has a high solid content and low viscosity, an antifouling paint composition having coating suitability can be obtained even without further adding the organic solvent to the resin composition when producing the antifouling paint composition. Further, in a case where an antifouling agent or the like is added, the antifouling agent can be mixed favorably without adding an organic solvent. Therefore, an antifouling paint composition having a low VOC content can be obtained.

[Antifouling paint composition]

The antifouling paint composition of the present invention is an antifouling paint composition containing the compound (A) and the vinyl-based copolymer (B).

The compound (A) contained in the antifouling paint composition of the present invention may be used alone or two or more kinds thereof may be used.

The content of the compound (A) in the antifouling paint composition of the present invention is 3% by mass or greater, with respect to the total amount of the antifouling paint composition.

The vinyl-based copolymer (B) contained in the antifouling paint composition of the present invention may be used alone or two or more kinds thereof may be used. The content of the vinyl-based copolymer (B) in the antifouling paint composition of the present invention is not particularly limited, but is preferably 5% by mass or greater, and more preferably 10% by mass or greater, with respect to the total amount of the resin composition. A ratio between the content of the compound (A) and the vinyl-based copolymer (B) in the antifouling paint composition is 0.1/99.9 to 70/30, and more preferably 90/10 to 40/60. In a case of the content ratio, excellent coatability is obtained with suppressed viscosity, and the water resistance and the antifouling property can be improved.

It is preferable that the antifouling paint composition of the present invention further include at least one selected from the group consisting of a compound that reacts with an acid, a basic compound, an acidic compound, and a dehydrating agent. As such, the storage stability of the resin composition and the antifouling paint composition containing this resin composition is improved.

In the vinyl-based copolymer (B), the structure (I) may become decomposed during storage. In a case where the structure (I) is decomposed, a carboxylic acid is generated. As such, the glass transition temperature of the vinyl-based copolymer (B) is increased, and the carboxylic acid and other components in the paint form a crosslinked structure and thus the viscosity of the solution of the vinyl-based copolymer (B) or the paint containing this solution may be increased. Further, generation of a free carboxylic acid results in degradation of the stability of dissolution in an organic solvent and the water resistance. In addition, by catalytically promoting the hydrolysis reaction of the generated carboxylic acid as an acid, decomposition of the structure (I) advances. In a case where the resin composition contains a compound that reacts with an acid, a carboxylic acid is captured by the compound that reacts with an acid at the time of decomposition of the structure (I) in the vinyl-based copolymer (B) to generate a carboxylic acid, and the storage stability is improved.

Further, in a high pH region or a low pH region, the storage stability is degraded due to the decomposition of the structure (I). In a high pH region, the storage stability is also degraded due to a decrease in the reactivity between a compound that reacts with an acid and carboxylic acid. The decomposition of the structure (I) is suppressed and the degradation of the storage stability can be suppressed by adjusting the pH of the resin composition through addition of a

basic compound or an acidic compound.

Further, moisture promotes decomposition (hydrolysis) of the structure (I). In a case where the resin composition contains a dehydrating agent, moisture in the resin composition is captured so that degradation of the storage stability can be suppressed.

Examples of the compound that reacts with an acid include the compound (Y3), a basic compound, and a compound containing an epoxy group. Examples of the compound (Y3) are the same as those exemplified as those of the compound (Y1) and the compound (Y2). The compound (Y3) is preferable as the compound that reacts with an acid.

Examples of the basic compound include dimethylamine, diethylamine, trimethylamine, triethylamine, aniline, and pyridine.

Examples of the compound containing an epoxy group include 2-ethyloxysilane, 2,3-dimethyloxysilane, 2,2-dimethyloxysilane, glycidyl (meth)acrylate, glycidyl $\alpha$-ethyl acrylate, and 3,4-epoxybutyl (meth)acrylate.

From the viewpoint of storage stability, the compound (Y3) is preferable as the compound that reacts with an acid. Among the examples of the compound, as the compound (Y3), 1-alkenyl alkyl ether in which X in Formula (31) represents -O- is preferable, and vinyl ethers such as butyl vinyl ether and isobutyl vinyl ether are more preferable from the viewpoint that the effect of improving the storage stability is further improved.

Examples of the basic compound used for adjusting the pH are the same as those exemplified as the basic compound described above.

Examples of the acidic compound include abietic acid, neoabietic acid, parastrinic acid, pimaric acid, isopimaric acid, levopimaric acid, dextropimaric acid, sandaracopimaric acid, acetic acid, propionic acid, butyric acid, lauric acid, stearic acid, linoleic acid, oleic acid, chloroacetic acid, and fluoroacetic acid.

Examples of the dehydrating agents include a silicate-based dehydrating agent, an isocyanate-based dehydrating agent, an ortho ester-based dehydrating agent, and an inorganic dehydrating agent. More specific examples thereof include methyl orthoformate, ethyl orthoformate, methyl orthoacetate, ortho-boronic esters, tetraethyl orthosilicate, anhydrous gypsum, calcined gypsum, and synthetic zeolite (molecular sieve). Among these, a molecular sieve is particularly preferable.

These additives may be used alone or in combination of two or more kinds thereof.

Examples of the combination of two or more kinds of the additives include a combination of the compound (Y3) and the dehydrating agent, a combination of the compound (Y3), the acidic compound, and the dehydrating agent, a combination of the compound (Y3), the basic compound, the acidic compound, and the dehydrating agent, and a combination of the basic compound and the dehydrating agent.

In a case where the antifouling paint composition contains the compound (Y3), the content of the compound (Y3) in the antifouling paint composition is preferably 20% by mole or greater, more preferably in a range of 30% to 1000% by mole, and still more preferably in a range of 40% to 800% by mole with respect to the amount of the structure (I) in the vinyl-based copolymer (B). In a case where the content of the compound (Y3) is in the above-described range, the effect of improving the storage stability is further improved.

In a case where the antifouling paint composition contains a basic compound or an acidic compound, from the viewpoint of storage stability, as the content of the basic compound or the acidic compound in the antifouling paint composition, the amount of the basic compound with a concentration set such that the pH to be measured in water is in a range of 2 to 12 is preferable and the amount of the basic compound with a concentration set such that the pH is in a range of 6 to 9 is more preferable.

Specifically, the pH to be measured in water refers to a value to be measured by adding the basic compound in water. The pH refers to a value at 23°C.

In a case where the antifouling paint composition contains a dehydrating agent, the content of the dehydrating agent in the resin composition is preferably in a range of 0.1% to 40% by mass and more preferably in a range of 1% to 20% by mass with respect to the total mass of the resin composition. In a case where the content of the dehydrating agent is greater than or equal to the lower limit of the above-described range, the storage stability is further improved. In a case where the content of the dehydrating agent is less than or equal to the upper limit of the above-described range, the dissolution stability is further improved.

<Silicone oil>

It is preferable that the antifouling paint composition of the present invention further contain a silicone oil. In a case where the antifouling paint composition contains the silicone oil, the antifouling property of a coating film is excellent.

Examples of the silicone oil include a straight silicone oil such as dimethyl silicone oil, methyl phenyl silicone oil, or methyl hydrogen silicone oil, and a modified silicone oil. A modified silicone oil refers to a silicone oil in which an organic group (hereinafter, also referred to as a "modification group") other than a methyl group and a phenyl group is introduced into some silicon atoms of a straight silicone oil. Examples of the modification group include a chlorophenyl group, a methylstyrene group, a long chain alkyl group (for example, an alkyl group having 2 to 18 carbon atoms), a polyether group,

a carbinol group, an aminoalkyl group, an epoxy group, and a (meth)acryloyl group. These silicone oils may be used alone or in combination of two or more kinds thereof. Among those described above, from the viewpoint of the antifouling property, a polyether-modified silicone oil having a polyether group as a modification group is preferable, as the silicone oil.

Examples of the silicone oil include "KF-96", "KF-50", "KF-54", "KF-56", and "KF-6016" (all manufactured by Shin-Etsu Chemical Co., Ltd.), "TSF451" (manufactured by Momentive Performance Materials Inc.), "Fluid47" (manufactured by (France) Rhone-Poulenc), and "SH200", "SH510", "SH550", "SH710", "DC200", "ST-114PA", and "FZ209" (all manufactured by Dow Corning Toray Co., Ltd.).

In a case where the antifouling paint composition contains a silicone oil, the content of the silicone oil in the resin composition is preferably in a range of 0.1% to 40% by mass and more preferably in a range of 1% to 20% by mass with respect to the total mass of the resin composition. In a case where the content of the silicone oil is greater than or equal to the lower limit of the above-described range, the antifouling property is further improved. In a case where the content of the silicone oil is less than or equal to the upper limit of the above-described range, the dissolution stability is further improved.

<Organic solvent>

It is preferable that the antifouling paint composition of the present invention contain an organic solvent. **In** a case where the antifouling paint composition contains an organic solvent, the coating suitability of the antifouling paint composition obtained by using the organic solvent, the water resistance of a coating film, and the film forming properties are further improved.

The organic solvent is not particularly limited as long as the vinyl-based copolymer (B) can be dissolved in the organic solvent, and examples thereof include a hydrocarbon-based solvent such as toluene or xylene; an ether-based solvent such as the compound (Y3) or propylene glycol monomethyl ether-2-acetate; a ketone-based solvent such as methyl isobutyl ketone; and an ester-based solvent such as n-butyl acetate. These can be used alone or in combination of two or more kinds thereof.

<Antifouling agent>

Examples of the antifouling agent include inorganic antifouling agents and organic antifouling agents, and one or more kinds can be suitably selected depending on the required performance.

Examples of the antifouling agent include a copper-based antifouling agent such as cuprous oxide, copper thiocyanate, or copper powder, compounds of other metals (lead, zinc, nickel, and the like), an amine derivative such as diphenylamine, a nitrile compound, a benzothiazole-based compound, a maleimide-based compound, and a pyridine-based compound. These antifouling agents may be used alone or in combination of two or more kinds thereof.

Examples of the antifouling agent include 4-bromo-2-(4-chlorophenyl)-5-(trifluoromethyl)-1H-pyrrole-3-carbonitrile, manganese ethylene bisdithiocarbamate, zinc dimethyl dithiocarbamate, 2-methylthio-4-t-butylamino-6-cyclopropylamino-s-triamine, 2,4,5,6-tetrachloroisophthalonitrile, N,N-dimethyldichlorophenylurea, zinc ethylene bisdithiocarbamate, copper rhodanide, 4,5-dichloro-2-n-octyl-4-isothiazolin-3-one, N-(fluorodichloromethylthio)phthalimide, N,N'-dimethyl-N'-phenyl-(N-fluorodichloromethylthio)sulfamide, 2-pyridinethiol-1-oxide zinc salt (also referred to as a "zinc pyrithione"), tetramethylthiuram disulfide, a Cu-10% Ni solid solution alloy, 2,4,6-trichlorophenylmaleimide 2,3,5,6-tetrachloro-4-(methylsulfonyl)pyridine, 3-iodine-2-propynyl butyl carbamate, diiodomethyl paratrisulfone, bisdimethyl dithiocarbamoyl zinc ethylene bisdithiocarbamate, phenyl(bispyridyl)bismuth dichloride, 2-(4-thiazolyl)-benzoimidazole, medetomidine, and pyridine triphenyl borane.

Among those described above, from the viewpoint of antifouling property, it is preferable that the antifouling agent contain at least one selected from the group consisting of cuprous oxide, pyridine triphenyl borane, 4,5-dichloro-2-n-octyl-4-isothiazolin-3-one, 4-bromo-2-(4-chlorophenyl)-5-(trifluoromethyl)-1H-pyrrole-3-carbonitrile (hereinafter, also referred to as an"antifouling agent (b1)"), and medetomidine.

In a case of combining cuprous oxide and the antifouling agent (b1), the blending ratio (mass ratio) of the cuprous oxide to the antifouling agent (b1) is preferably in a range of 80/20 to 99/1, and more preferably 90/10 to 99/1.

At least one selected from the group consisting of cuprous oxide, pyridinetriphenylborane, 4,5-dichloro-2-n-octyl-4-isothiazolin-3-one, the antifouling agent (b 1), and medetomidine may be combined with other antifouling agents.

In a case where the antifouling paint composition contains an antifouling agent, the content of the antifouling agent in the antifouling paint composition is not particularly limited, but is preferably in a range of 10 to 200 parts by mass and more preferably in a range of 50 to 150 parts by mass with respect to 100 parts by mass of the copolymer (A). In a case where the content of the antifouling agent is greater than or equal to the lower limit of the above-described range, the antifouling effect of a coating film is greatly improved. In a case where the content of the antifouling agent is less than or equal to the upper limit of the above-described range, the physical properties of the coating film are improved.

<Other components>

Examples of other components include thermoplastic resins or the like other than the vinyl-based copolymer (B). It is preferable that the antifouling paint composition of the present aspect contain the thermoplastic resin other than the vinyl-based copolymer (B). In a case where the antifouling paint composition contains a thermoplastic resin other than the vinyl-based copolymer (B), the physical properties of the coating film such as crack resistance and water resistance are improved.

Examples of the thermoplastic resins other than the vinyl-based copolymer (B) include alkyd resin; polyester resin and chlorinated paraffin; wax; fats and oils that are solid at room temperature, other waxes, fats and oils that are liquid at room temperature, such as castor oil, and refined products thereof; vaseline; liquid paraffin; and rosin, hydrogenated rosin, naphthenic acid, fatty acids, and divalent metal salts thereof. Examples of the waxes include waxes derived from animals such as beeswax; waxes derived from plants; semi-synthetic waxes such as amide-based waxes; and synthetic waxes such as polyethylene oxide-based waxes. These thermoplastic resins may be used alone or in combination of two or more kinds thereof.

From the viewpoint of functioning as a plasticizer and having an effect of improving the crack resistance and the peeling resistance of the coating film, the chlorinated paraffin is preferable.

From the viewpoints of functioning as an antisettling agent and the antisagging agent and having an effect of improving the storage stability or the pigment dispersibility of the antifouling paint composition, organic waxes such as semi-synthetic wax and synthetic wax are preferable, and polyethylene wax, oxidized polyethylene wax, and polyamide wax are more preferable.

The content of the thermoplastic resin other than the vinyl-based copolymer (B) in the antifouling paint composition is not particularly limited, but is preferably in a range of 0.1 to 50 parts by mass and more preferably in a range of 0.1 to 10 parts by mass with respect to 100 parts by mass of the vinyl-based copolymer (B). In a case where the content of the thermoplastic resin other than the vinyl-based copolymer (B) is greater than or equal to the lower limit of the above-described range, the physical properties of the coating film such as crack resistance or water resistance are improved.

**In** a case where the content thereof is less than or equal to the upper limit of the above-described range, the hydrolyzability is improved.

**In** order to impart lubricity to the surface of the coating film and preventing adhesion of organisms, the antifouling paint composition of the present invention may contain a silicon compound such as dimethylpolysiloxane (excluding a silicone oil), a fluorine-containing compound such as fluorinated hydrocarbon, or the like.

The antifouling paint composition of the present invention may contain various pigments, antifoaming agents, leveling agents, pigment dispersants (such as antisettling agents), antisagging agents, matting agents, ultraviolet absorbing agents, antioxidants, heat resistance improvers, slipping agents, preservatives, plasticizers, and viscosity control agents.

Examples of the pigments include zinc oxide, talc, silica, barium sulfate, potassium feldspar, aluminum hydroxide, magnesium carbonate, mica, carbon black, red iron oxide, titanium oxide, phthalocyanine blue, kaolin, and gypsum. In particular, zinc oxide or talc are preferable.

Examples of the antisettling agent and the antisagging agent other than the thermoplastic resin include a bentonite-based agent, a fine powder silica-based agent, a stearate salt, a lecithin salt, and an alkyl sulfonate.

Examples of the plasticizer other than the thermoplastic resin include a phthalic acid ester-based plasticizer such as dioctyl phthalate, dimethyl phthalate, dicyclohexyl phthalate, or diisodecyl phthalate; an aliphatic dibasic acid ester-based plasticizer such as isobutyl adipate or dibutyl sebacate; a glycol ester-based plasticizer such as diethylene glycol dibenzoate or pentaerythritol alkyl ester; a phosphoric acid ester-based plasticizer such as tricresyl phosphate (TCP), triaryl phosphate, or trichloroethyl phosphate; an epoxy-based plasticizer such as epoxy soybean oil or octyl epoxy stearate; an organic tin plasticizer such as dioctyl tin laurate or dibutyl tin laurate; and trioctyl trimellitate, and triacetylene. In a case where the antifouling paint composition contains a plasticizer, the crack resistance and the peeling resistance of the coating film can be improved. Among those described above, TCP is preferable as a plasticizer.

From the viewpoint of reducing the VOC content in the antifouling paint composition, the content of the organic solvent in the antifouling paint composition of the present invention is preferably 60% by mass or less, more preferably 50% by mass or less, and still more preferably 45% by mass or less, and particularly preferably 40% by mass or less with respect to the total amount of the resin composition.

The content of the organic solvent is preferably set to an amount such that the viscosity of the antifouling paint composition to be measured at 25°C using a B type viscometer is less than or equal to the preferable upper limit described below, and varies depending on the weight-average molecular weight of the vinyl-based copolymer (B), the glass transition temperature thereof, the presence of a crosslinked structure, and the like, but is preferably 15% by mass or greater and more preferably 20% by mass or greater.

Further, the compound (Y3) can function as an organic solvent. Therefore, in a case where the resin composition

contains the compound (Y3), the content of the compound (Y3) is included in the content of the organic solvent.

<Viscosity>

In a case where the antifouling paint composition of the present invention contains a solvent, the viscosity of the resin composition to be measured at 25°C using a B type viscometer (hereinafter, also referred to as a "B type viscosity") is preferably less than 5000 mPa·s, more preferably less than 4000 mPa·s, still more preferably less than 3000 mPa·s, and particularly preferably less than 2000 mPa·s.

The viscosity of the antifouling paint composition can be adjusted depending on the solid content of the resin composition (the content of the vinyl-based copolymer (B) and other components), the weight-average molecular weight of the vinyl-based copolymer (B), the glass transition temperature, and the presence of the crosslinked structure. For example, the viscosity tends to be lowered as the solid content, particularly the content of the vinyl-based copolymer (B), decreases. In addition, the viscosity tends to be lowered as the weight-average molecular weight of the vinyl-based copolymer (B) decreases or the glass transition temperature decreases.

The solid content of the antifouling paint composition of the present invention is preferably in a range of 55% to 100% by mass, more preferably in a range of 60% to 90% by mass, and still more preferably in a range of 65% to 80% by mass.

In a case where the solid content of the antifouling paint composition is greater than or equal to the lower limit of the above-described range, the VOC content is sufficiently decreased. In a case where the solid content is less than or equal to the upper limit of the above-described range, the viscosity of the antifouling paint composition is easily lowered.

The B type viscosity of the antifouling paint composition of the present invention measured at 25°C is preferably less than 5000 mPa·s, preferably less than 3000 mPa·s, and more preferably less than 1000 mPa·s. In a case where the viscosity of the antifouling paint composition is less than or equal to the above-described upper limit, the antifouling paint composition is easily applied.

The lower limit of the B type viscosity of the antifouling paint composition is not particularly limited, but is preferably 100 mPa·s or greater from the viewpoint of the physical properties of the coating film.

The viscosity of the antifouling paint composition can be adjusted depending on the viscosity of the resin composition, the amount of the organic solvent to be added to the resin composition, and the like.

The antifouling paint composition of the present invention can be used for forming a coating film (antifouling coating film) on a surface of a base material, for example, underwater structures such as ships, various fishing nets, port facilities, oil fences, bridges, and submarine bases.

The coating film formed of the antifouling paint composition of the present invention can be formed on a surface of a base material directly or through a ground coating film.

As the ground coating film, a wash primer, a chlorinated rubber-based primer, or an epoxy-based primer, or an intermediate paint can be used for forming the coating film.

The coating film can be formed using a known method. For example, the surface of the base material or the ground coating film on the base material can be coated with the antifouling paint composition by means of brush coating, spray coating, roller coating, or dip coating and dried, thereby forming a coating film.

The coating amount of the antifouling paint composition can be typically set such that the thickness of the dried coating film is in a range of 10 to 400 μm.

The coating film can be typically dried at room temperature and may be dried by being heated as necessary.

[Examples]

Hereinafter, the present invention will be described in more detail based on examples and comparative examples, but the present invention is not limited to these examples. Further, parts in the examples indicate parts by mass.

The evaluation in the examples was performed by the methods shown below.

(Solid content (heating residue))

0.50 g of a measured sample (the resin composition or the antifouling paint composition) are weighed on an aluminum dish (measured mass), 3 mL of toluene was added thereto with a dropper to spread uniformly on the bottom of the dish, and the sample was heated and dissolved on a water bath at 70°C to 80°C and evaporated to dryness. Thereafter, drying was performed using a hot air dryer at 105°C for 2 hours. The solid content (heating residue) was determined using the following equation based on the mass (measured mass) of the sample and the mass (mass after drying) of the sample after the drying.

$$\text{Solid content (\% by mass)} = \text{mass after drying/measured mass} \times 100$$

(B type viscosity)

The viscosity of the sample was measured using a B type viscometer at 25°C, and the value was shown as the B type viscosity.

(Gardner viscosity)

A sample was put into a dried Gardner bubble viscosity tube (hereinafter, also simply referred to as a viscosity tube) up to an indication line of the viscosity tube, and the tube was sealed with a cork stopper. The viscosity tube with the collected sample was vertically immersed in a constant temperature water bath whose temperature was adjusted to a predetermined temperature (25.0 ± 0.1°C) for 2 hours so that the temperature of the sample was constant, a viscosity tube serving as a reference tube and the viscosity tube to which the sample was added were allowed to rotate by 180° simultaneously, and the viscosity (Gardner viscosity) was determined by comparing the bubble increase rate of the sample in the viscosity tube with that in the reference tube.

(Weight-average molecular weight (Mw) and number-average molecular weight (Mn))

The weight-average molecular weight (Mw) and number-average molecular weight (Mn) of the polymer were measured using gel permeation chromatography (GPC) (manufactured by Tosoh corporation, HLC-8220). As the column, TSK gel α-M (manufactured by Tosoh Corporation, 7.8 mm × 30 cm) and TSK guard column α (manufactured by Tosoh corporation, 6.0 mm × 4 cm) were used. A calibration curve was prepared using F288/F1/28/F80/F40/F20/F2/A1000 (manufactured by Tosoh Corporation, standard polystyrene) and styrene monomer.

(Coating suitability)

The smoothness of the coating film after coating was visually confirmed, and the coating suitability was evaluated according to the following criteria.

◦: Coating film was smooth.

△: Streaks remained partially on the coating film.

×: Streaks remained on the coating film.

(Paint viscosity change rate)

The B type viscosity (B type viscosity before storage) (mPa·s) of the produced antifouling paint composition was measured. The antifouling paint composition was put into a 150 ml glass bottle and stored at 40°C for 30 days. Thereafter, the B type viscosity (B type viscosity after storage at 40°C for 30 days) (mPa·s) of the antifouling paint composition was measured, and the paint viscosity change rate (%) was calculated by the following equation.

Paint viscosity change rate (%) = B type viscosity after storage at 40°C for 30 days/ B type viscosity before storage × 100

(Water resistance of coating film of antifouling paint composition)

A hard vinyl chloride plate having a size of 50 mm × 50 mm × 2 mm was coated with the antifouling paint composition using an applicator such that the thickness of the dried film was set to 120 μm, and the film was dried to form a coating film, thereby obtaining a test plate. This test plate was attached to a rotating drum installed in seawater and rotated at a peripheral speed of 7.7 m/s (15 knots). The state was maintained for 6 months, and the surface of the coating film after 6 months was observed. The evaluation was performed according to the following criteria.

◎: No crack or peeling was observed.

◦: Cracks were partially observed.

△: Cracks and peeling were observed in some parts.

×: Cracks and peeling were observed on the entire surface.

(Static antifouling property)

A sandblasted steel sheet to which an anticorrosive paint had been applied in advance was coated with the antifouling paint composition using a brush such that the thickness of the dried film was set to 200 to 300 μm, and the film was dried to form a coating film, thereby obtaining a test plate. After allowing this test plate to stand in Mikawa Bay for 6 months, a ratio of the area where marine organisms adhered to the total area of the coating film (the area where marine organisms adhered) was evaluated, and the static antifouling property was determined according to the following criteria.

◎: Area where seawater organisms adhered was 10% or less.

◦: Area where seawater organisms adhered was more than 10% and 20% or less.

△: Area where seawater organisms adhered was more than 20% and 40% or less.

×: Area where seawater organisms adhered was more than 40%.

(Coating film consumption degree test)

A hard vinyl chloride plate having a size of 50 mm × 50 mm × 2 mm was coated with the antifouling paint composition using an applicator such that the thickness of the dried film was set to 120 μm, and the film was dried to form a coating film, thereby obtaining a test plate. This test plate was attached to a rotating drum installed in seawater and rotated at a peripheral speed of 7.7 m/s (15 knots). This state was maintained for 6 months, the film thickness (μm) of the coating film after 6 months was measured, and the film thickness depletion per month was defined as the depletion degree (μm/M).
The meanings of the abbreviations used in each of the following examples are as follows.

IBEMA: 1-isobutoxyethyl methacrylate (synthetic product obtained by synthesis in Production Example M1 described below)
MMA: Methyl methacrylate
EA: Ethyl acrylate
BA: Butyl acrylate
CHMA: Cyclohexyl methacrylate
2-MTMA: 2-Methoxyethyl methacrylate
MAA: Methacrylic acid.
TIPSA: Triisopropylsilyl acrylate
TIPSMA: Triisopropylsilyl methacrylate.
AMBN: 2,2'-azobis(2-methylbutyronitrile)
NOFMER MSD: Trade name, manufactured by NOF Corporation, α-methylstyrene dimer
Additive (a): DISPARLON (registered trademark) 4200-20 (manufactured by Kusumoto Chemicals, Ltd., oxidized polyethylene wax)
Additive (b): DISPARLON A603-20X (manufactured by Kusumoto Chemicals, Ltd., polyamide wax)
Additive (c): TOYOPARAX (registered trademark) 150 (manufactured by Tosoh corporation, chlorinated paraffin)
KF-6016: trade name, manufactured by Shin-Etsu Chemical Co., Ltd., polyether-modified silicone oil
Antifouling agent (1): 4-bromo-2- (4-chlorophenyl) -5- (trifluoromethyl) -1H-pyrrole-3-carbonitrile
Antifouling agent (2): 4,5-dichloro-2-n-octyl-4-isothiazolin-3-one (manufactured by ROHM and Haas, trade name: Sea Nine 211)

[Production Example M1]

90.1 parts (0.9 mol) of isobutyl vinyl ether, 0.14 parts of hydroquinone, and 0.28 parts of phenothiazine were stirred at room temperature and mixed to obtain a uniform solution. 51.7 parts (0.6 mol) of methacrylic acid was added dropwise while air (10 ml/min) was blown such that the temperature of the reaction solution was maintained at 60°C or lower. After dropwise addition, the temperature of the reaction solution was increased to 80°C and the reaction was continued for 6 hours. 158.7 parts (1.8 mol) of t-butyl methyl ether was added to the reaction solution, the solution was mixed, and the organic phase was washed once using 200 parts of a 20 mass% potassium carbonate aqueous solution.

0.03 parts of 4-benzoyloxy-2,2,6,6-tetramethylpiperidine-N-oxyl was added to the organic phase so that the low boiling content was distilled off using an evaporator. The resulting residue was distilled off under reduced pressure, thereby obtaining 97.5 (0.52 mol) of 1-isobutoxyethyl methacrylate (IBEMA) having a boiling point of 60°C/3 torr.

[Production Example A-1]

A reaction container provided with a stirrer, a temperature adjuster, and a dropping device was charged with a liquid in which 100 parts of rosin was dissolved in 100 parts of xylene, and the mixture was heated to 120°C while being stirred. 66.2 parts of isobutyl vinyl ether was added thereto for 30 minutes, and after stirring for 6 hours, a compound A-1 having a solid content of 48.8% by mass was obtained.

[Production Example A-2]

A reaction container provided with a stirrer, a temperature adjuster, and a dropping device was charged with a liquid in which 100 parts of rosin was dissolved in 100 parts of xylene, and the mixture was heated to 120°C while being stirred. 103.4 parts of 2-ethylhexyl vinyl ether was added thereto for 30 minutes, and after stirring for 6 hours, a compound A-2 having a solid content of 49.2% by mass was obtained.

[Production Example A-3]

A reaction container provided with a stirrer, a temperature adjuster, and a dropping device was charged with a liquid in which 100 parts of versatic acid was dissolved in 100 parts of xylene, and the mixture was heated to 120°C while being stirred. 117 parts of isobutyl vinyl ether was added thereto for 30 minutes, and after stirring for 6 hours, a compound A-3 having a solid content of 49.0% by mass was obtained.

[Production Example B-1]

A reaction container provided with a stirrer, a temperature adjuster, and a dropping device was charged with 50 parts of industrial xylene, and the mixture was heated to 90°C while being stirred. Subsequently, a mixture including 25 parts of monomer (M1), 24 parts of MMA, 36 parts of EA, 15 parts of MTMA, and 1.9 parts of AMBN as an initiator was added dropwise from a dropping funnel at a constant rate over 4 hours. Thirty minutes after the completion of the the dropwise addition, 9.9 parts of xylene and 2.0 parts of AMBN were added dropwise at a constant rate over 30 minutes. After further stirring the mixture for 2 hours, 6.7 parts of isobutyl vinyl ether was added to obtain a resin composition B-1 containing the vinyl-based copolymer (B) having a solid content of 60% by mass.

[Production Examples B-2 to B-5]

The types and charging amounts of monomers and initiators, and the types and amounts of additives to be added after polymerization were set as shown in Table 1, the same as in Production Example B-1 was applied except that the amount of xylene after completion of the dropwise addition was adjusted according to the amount of the additive so that the theoretical solid content was 60% by mass. Therefore, resin compositions B-2 to B-5 containing the vinyl-based copolymer (B) were produced.

[Production Example B-6]

A reaction container provided with a stirrer, a temperature adjuster, and a dropping device was charged with 76.7 parts of propylene glycol monomethyl ether acetate, and the mixture was heated to 90°C while being stirred. Subsequently, a mixture including 11.6 parts of MAA, 24 parts of MMA, 36 parts of EA, 15 parts of MTMA, and 1.9 parts of AMBN as an initiator was added dropwise from a dropping funnel at a constant rate over 4 hours. After 30 minutes from the completion of the the dropwise addition, 9.9 parts of xylene and 2.0 parts of AMBN were added dropwise at a constant rate over 30 minutes. Furthermore, after stirring the mixture for 2 hours, the temperature was raised to 110°C, 26.8 parts of isobutyl vinyl ether was added dropwise at a constant rate over 30 minutes, and then the mixture was further stirred for 6 hours. Accordingly, the carboxy group in the copolymer was reacted with the isobutyl vinyl ether to obtain a resin composition B-6 containing the vinyl-based copolymer (B) having a solid content of 50.1% by mass.

Characteristics (solid content (% by mass), B-type viscosity, Gardner viscosity, and the number-average molecular weight (Mn) and the weight-average molecular weight (Mw) of the copolymer contained in each resin composition) of the obtained resin compositions B-1 to B-6 are listed in Table 1 as results.

[Table 1]

| Resin composition | | B-1 | B-2 | B-3 | B-4 | B-5 | B-6 |
|---|---|---|---|---|---|---|---|
| Monomer (m0) | MAA | - | - | - | - | - | 11.6 |
| Monomer (m1) | IBEMA | 25 | - | 25 | 10 | 10 | - |
| Monomer (m2) | TIPSA | - | 30.5 | - | - | - | - |
| | TIPSMA | - | - | - | - | 15 | - |
| Monomer (m4) | MMA | 24 | 35.5 | 10.5 | 27 | 14 | 24 |
| | EA | 36 | 19 | 19.5 | 48 | 46 | 36 |
| | CHMA | - | - | 45 | - | - | - |
| | BA | - | - | - | - | - | - |
| | 2-MTMA | 15 | 15 | - | 15 | 15 | 15 |
| Total | | 100 | 100 | 100 | 100 | 100 | 86.6 |
| Compound (Y3) | isobutyl vinyl ether | 6.7 | - | 6.7 | 6.7 | 6.7 | 26.8 |
| Content of the compound (Y3) with respect to amount of structure (I) (% by mol) | | 50 | - | 50 | 167 | 167 | 100 |
| Initiator | AMBN | 1.9 | 1.8 | 1.9 | 2.1 | 2.1 | 1.9 |
| Chain transfer agent | NOFMER MSD | - | - | - | - | - | |
| Characteristic value | Solid content (% by mass) | 60.2 | 60.8 | 60.8 | 60.9 | 60.9 | 50.1 |
| | B type viscosity (mPa·s) (25°C) | 1070 | 1020 | 1050 | 960 | 960 | 240 |
| | Gardner viscosity (25°C) | W | W- | W- | VW | VW | IJ |
| | Number-average molecular weight Mn | 7100 | 6500 | 7200 | 7700 | 7700 | 6700 |
| | Weight-average molecular weight Mw | 20200 | 18600 | 21000 | 21400 | 21400 | 22000 |

In Table 1, the numerical values described in columns of Monomer and Initiator indicate the charged amount (part(s)).

[Examples 1 to 8 and Comparative Examples 1 to 4]

Respective components were mixed using a rocking shaker, according to formulations shown in Table 2 to obtain an antifouling paint composition.

Table 2 shows evaluation results of paint properties (solid content and B type viscosity), the coating suitability, the paint viscosity change rate, and the coating film performance (static antifouling property, water resistance, and coating film consumption degree test) of the obtained antifouling paint compositions.

[Table 2-1]

| Table 2 (1/3) | | | | | | |
|---|---|---|---|---|---|---|
| | | | Example 1 | Example 2 | Example 3 | Example 4 |
| | Compound (A) | A-1 solution | 15.1 | 30.2 | 15.1 | 15.1 |
| | | A-2 solution | - | - | - | - |
| | | A-3 solution | - | - | - | - |
| | Rosin (xylene 50% solution) | | - | - | - | - |

(continued)

| Table 2 (1/3) | | | | | | |
|---|---|---|---|---|---|---|
| | | | Example 1 | Example 2 | Example 3 | Example 4 |
| Composition | Resin composition | B-1 solution | 37.8 | 25.2 | - | - |
| | | B-2 solution | - | - | 37.8 | - |
| | | B-3 solution | - | - | - | 37.8 |
| | | B-4 solution | - | - | - | - |
| | | B-5 solution | - | - | - | - |
| | | B-6 solution | - | - | - | - |
| | Pigment | Talc | 16.5 | 16.5 | 16.5 | 16.5 |
| | | Titanium oxide | 4.7 | 4.7 | 4.7 | 4.7 |
| | Antifouling agent | Cuprous oxide | 106 | 106 | 106 | 106 |
| | | Copper pyrithione | 2 | 2 | 2 | 2 |
| | | Medetomidine | - | - | - | - |
| | | Antifouling agent (1) | - | - | - | - |
| | | Antifouling agent (2) | - | - | - | - |
| | Dehydrating agent | Molecular sieve 4A | 1 | 1 | 1 | 1 |
| | Additive | Additive (a) | 6 | 6 | 6 | 6 |
| | | Additive (b) | 5 | 5 | 5 | 5 |
| | | Additive (c) | - | - | - | - |
| | | KF-6016 | - | - | - | - |
| | Solvent | Xylene | 21.1 | 18.6 | 21.1 | 21.1 |
| Paint property | | Solid content (% by mass) | 75.6 | 75.7 | 75.5 | 75.8 |
| | | B type viscosity (mPa·s) (25°C) | 2500 | 1410 | 1680 | 1860 |
| | | Coating suitability | ○ | ○ | ○ | ○ |
| | | Paint viscosity change rate (%) | 105 | 105 | 101 | 105 |
| Coating film performance | | Static antifouling property | ◎ | ◎ | ◎ | ◎ |
| | | Water resistance | ◎ | ◎ | ◎ | ◎ |
| | | Coating film consumption degree | 7.7 | 5.6 | 4.1 | 3 |

[Table 2-2]

| Table 2 (2/3) | | | | | | |
|---|---|---|---|---|---|---|
| | | | Example 5 | Example 6 | Example 7 | Example 8 |
| | Compound (A) | A-1 solution | 15.1 | 15.1 | - | - |
| | | A-2 solution | - | - | 15.1 | - |
| | | A-3 solution | - | - | - | 15.1 |
| | Rosin (xylene 50% solution) | | - | - | - | - |

(continued)

| Table 2 (2/3) | | | | | | |
|---|---|---|---|---|---|---|
| | | | Example 5 | Example 6 | Example 7 | Example 8 |
| Composition | Resin composition | B-1 solution | - | - | - | 37.8 |
| | | B-2 solution | - | - | - | - |
| | | B-3 solution | - | - | - | - |
| | | B-4 solution | 37.8 | - | - | - |
| | | B-5 solution | - | 37.8 | - | - |
| | | B-6 solution | - | - | 37.8 | - |
| | Pigment | Talc | 16.5 | 16.5 | 16.5 | 16.5 |
| | | Titanium oxide | 4.7 | 4.7 | 4.7 | 4.7 |
| | Antifouling agent | Cuprous oxide | 106 | 101 | 106 | 106 |
| | | Copper pyrithione | 2 | 2 | 2 | 2 |
| | | Medetomidine | - | 1 | - | - |
| | | Antifouling agent (1) | - | 1 | - | - |
| | | Antifouling agent (2) | - | 1 | - | - |
| | Dehydrating agent | Molecular sieve 4A | 1 | 1 | 1 | 1 |
| | Additive | Additive (a) | 6 | 6 | 6 | 6 |
| | | Additive (b) | 5 | 5 | 5 | 5 |
| | | Additive (c) | - | 1 | - | - |
| | | KF-6016 | - | 1 | - | - |
| | Solvent | Xylene | 21.1 | 21.1 | 17.3 | 21.1 |
| Paint property | | Solid content (% by mass) | 75.6 | 75.6 | 75.7 | 75.4 |
| | | B type viscosity (mPa·s) (25°C) | 2650 | 1820 | 2100 | 1800 |
| | | Coating suitability | ○ | ○ | ○ | ○ |
| | | Paint viscosity change rate (%) | 108 | 110 | 110 | 101 |
| Coating film performance | | Static antifouling property | ◎ | ◎ | ◎ | ○ |
| | | Water resistance | ◎ | ◎ | ◎ | ○ |
| | | Coating film consumption degree (μm/M) | 5.2 | 4.4 | 3.8 | 3.3 |

[Table 2-3]

| Table 2 (3/3) | | | | | | |
|---|---|---|---|---|---|---|
| | | | Comparative Example 1 | Comparative Example 2 | Comparative Example 3 | Comparative Example 4 |
| | Compound (A) | A-1 solution | - | - | - | - |
| | | A-2 solution | - | - | - | - |
| | | A-3 solution | - | - | - | - |
| | Rosin (xylene 50% solution) | | - | - | 15.1 | 15.1 |

(continued)

Table 2 (3/3)

| | | | Comparative Example 1 | Comparative Example 2 | Comparative Example 3 | Comparative Example 4 |
|---|---|---|---|---|---|---|
| Composition | Resin composition | B-1 solution | 50.4 | - | 37.8 | - |
| | | B-2 solution | - | 50.4 | - | 37.8 |
| | | B-3 solution | - | - | - | - |
| | | B-4 solution | - | - | - | - |
| | | B-5 solution | - | - | - | - |
| | | B-6 solution | - | - | - | - |
| | Pigment | Talc | 16.5 | 16.5 | 16.5 | 16.5 |
| | | Titanium oxide | 4.7 | 4.7 | 4.7 | 4.7 |
| | Antifouling agent | Cuprous oxide | 106 | 106 | 106 | 106 |
| | | Copper pyrithione | 2 | 2 | 2 | 2 |
| | | Medetomidine | - | - | - | - |
| | | Antifouling agent (1) | - | - | - | - |
| | | Antifouling agent (2) | - | - | - | - |
| | Dehydrating agent | Molecular sieve 4A | 1 | 1 | 1 | 1 |
| | Additive | Additive (a) | 6 | 6 | 6 | 6 |
| | | Additive (b) | 5 | 5 | 5 | 5 |
| | | Additive (c) | - | - | - | - |
| | | KF-6016 | - | - | - | - |
| | Solvent | Xylene | 23.5 | 23.5 | 21.1 | 21.1 |
| Paint property | | Solid content (% by mass) | 75.5 | 75.5 | 75.3 | 75.5 |
| | | B type viscosity (mPa·s) (25°C) | 3540 | 3100 | 5200 | 4550 |
| | | Coating suitability | Δ | Δ | Δ | Δ |
| | | Paint viscosity change rate (%) | 103 | 102 | 580 | 130 |
| Coating film performance | | Static antifouling property | Δ | Δ | ◎ | ◎ |
| | | Water resistance | ◎ | ◎ | × | × |
| | | Coating film consumption degree (μm/M) | 3.4 | 2.5 | 1.4 | 2.2 |

In Table 2, the numerical values described in column of Composition indicate the blending amount (part(s)). The blending amount of the resin composition is the total amount of the resin composition.

The antifouling paint compositions of Examples 1 to 8 had a low viscosity even with a high solid content and had good coating suitability.

In addition, the coating films using the antifouling paint compositions of Examples 1 to 8 had excellent static antifouling property and water resistance. In addition, this coating film had an acceptable consumption degree.

On the other hand, Comparative Examples 1 to 4 using the antifouling paint composition not containing the compound (A) showed a solid content equivalent to that of the resin composition having the compound (A) and showed high viscosity with a high B type viscosity value. Due to the high viscosity, the antifouling paint composition has poor coating suitability, and is not suitable for low VOC paints.

[Industrial Applicability]

An antifouling paint composition of the present invention can be suitably used for antifouling paint applications.

## Claims

1. An antifouling paint composition, comprising:

   at least one compound (A) selected from the group consisting of compounds represented by Formula (1), Formula (2), and Formula (3); and
   a vinyl-based copolymer (B), wherein the content of the compound (A) in the antifouling paint composition is 3% by mass or greater, and the mass ratio of the content of the compound (A) to the vinyl-based copolymer (B) in the antifouling paint composition is 0.1/99.9 to 70/30,

   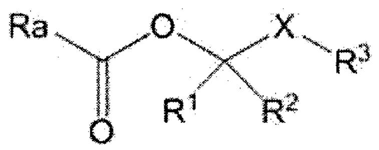
   (1)
   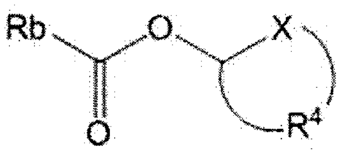
   (2)
   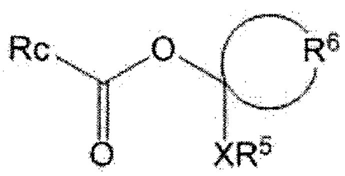
   (3)

   (in the formulae, Ra, Rb, and Rc represent a hydrocarbon group having 1 to 40 carbon atoms; X represents -O-, -S-, or $-NR^{14}-$, where $R^{14}$ represents a hydrogen atom or an alkyl group; $R^1$ and $R^2$ each represents a hydrogen atom or an alkyl group having 1 to 10 carbon atoms; $R^3$ and $R^5$ each represents an alkyl group having 1 to 20 carbon atoms, a cycloalkyl group, or an aryl group; and $R^4$ and $R^6$ each represents an alkylene group having 1 to 10 carbon atoms).

2. The antifouling paint composition according to Claim 1, wherein Ra, Rb, or Rc of the compound (A) is a cyclic hydrocarbon residue derived from rosin.

3. The antifouling paint composition according to Claim 1 or 2,

   wherein the vinyl-based copolymer (B) is a (meth)acrylic copolymer including at least one constituent unit selected from the group consisting of a constituent unit (u1) having at least one structure (I) represented by Formula (4), Formula (5), or Formula (6), a constituent unit (u2) containing a triorganosilyloxycarbonyl group, and a constituent unit (u3) having at least one structure (III) represented by Formula (7) or Formula (8),

   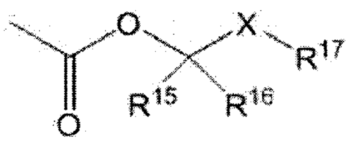
   (4)
   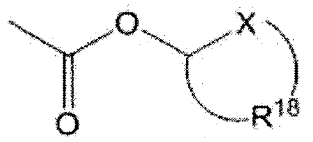
   (5)
   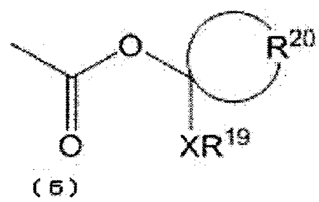
   (6)

   (in the formulae, X represents -O-, -S-, or $-NR^{21}-$, where $R^{21}$ represents a hydrogen atom or an alkyl group; $R^{15}$ and $R^{16}$ each represents a hydrogen atom or an alkyl group having 1 to 10 carbon atoms; $R^{17}$ and $R^{19}$ each

represents an alkyl group having 1 to 20 carbon atoms, a cycloalkyl group, or an aryl group; and $R^{18}$ and $R^{20}$ each represents an alkylene group having 1 to 10 carbon atoms),

-COO-M-OCO ··· (7)

-COO-M-$R^{13}$ ··· (8)

(in the formulae, M represents Zn, Cu, Mg, or Ca; and $R^{13}$ represents an organic acid residue other than a (meth) acryloyloxy group).

**4.** The antifouling paint composition according to any one of Claims 1 to 3, further comprising:
at least one selected from the group consisting of a compound that reacts with an acid, a basic compound, an acidic compound, and a dehydrating agent.

**5.** The antifouling paint composition according to Claim 4,

wherein the compound that reacts with an acid is at least one compound (Y3) selected from the group consisting of compounds represented by Formula (31), Formula (32), and Formula (33),

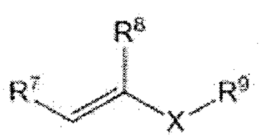
(31)

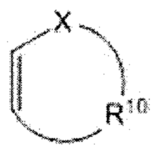
(32)

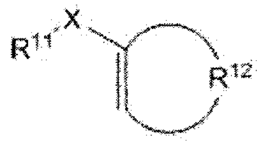
(33)

(in the formulae, X represents -O-, -S-, or -$NR^{21}$-, where $R^{21}$ represents a hydrogen atom or an alkyl group; $R^7$ represents a hydrogen atom or an alkyl group having 1 to 9 carbon atoms; $R^8$ represents a hydrogen atom or an alkyl group having 1 to 10 carbon atoms; $R^9$ and $R^{11}$ each represents an alkyl group having 1 to 20 carbon atoms, a cycloalkyl group, or an aryl group; $R^{10}$ represents a single bond or an alkylene group having 1 to 9 carbon atoms; and $R^{12}$ represents an alkylene group having 1 to 9 carbon atoms).

**6.** The antifouling paint composition according to any one of Claims 1 to 5, further comprising an antifouling agent.

**7.** The antifouling paint composition according to Claim 6,
wherein the antifouling paint composition contains, as the antifouling agent, at least one selected from the group consisting of cuprous oxide, pyridine triphenyl borane, 4,5-dichloro-2-n-octyl-4-isothiazolin-3-one, 4-bromo-2-(4-chlorophenyl)-5-(trifluoromethyl)-1H-pyrrole-3-carbonitrile, and medetomidine.

## Patentansprüche

**1.** Antifouling-Farbzusammensetzung, umfassend:

mindestens eine Verbindung (A), ausgewählt aus der Gruppe, bestehend aus durch Formel (1), Formel (2) und Formel (3) dargestellten Verbindungen, und
ein Copolymer auf Vinylbasis (B), wobei der Gehalt der Verbindung (A) in der Antifouling-Farbzusammensetzung 3 Masse-% oder größer ist und ein Massenverhältnis des Gehalts der Verbindung (A) zu dem Copolymer auf Vinylbasis (B) in der Antifouling-Farbzusammensetzung 0,1/99,9 bis 70/30 beträgt,

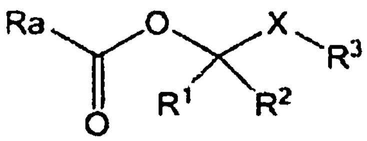
(1)

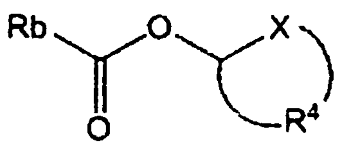
(2)

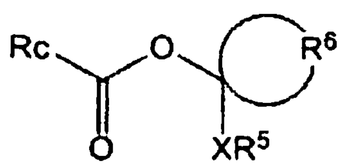
(3)

(in den Formeln stellen Ra, Rb und Rc eine Kohlenwasserstoffgruppe mit 1 bis 40 Kohlenstoffatomen dar, X stellt -O-, -S- oder -$NR^{14}$- dar, worin $R^{14}$ ein Wasserstoffatom oder eine Alkylgruppe darstellt, $R^1$ und $R^2$ stellen jeweils ein Wasserstoffatom oder eine Alkylgruppe mit 1 bis 10 Kohlenstoffatomen dar, $R^3$ und $R^5$ stellen jeweils eine Alkylgruppe mit 1 bis 20 Kohlenstoffatomen, eine Cycloalkylgruppe oder eine Arylgruppe dar und $R^4$ und $R^6$ stellen jeweils eine Alkylengruppe mit 1 bis 10 Kohlenstoffatomen dar).

**2.** Antifouling-Farbzusammensetzung gemäß Anspruch 1, wobei Ra, Rb oder Rc der Verbindung (A) ein von Kolophonium abgeleiteter cyclischer Kohlenwasserstoffrest ist.

**3.** Antifouling-Farbzusammensetzung gemäß Anspruch 1 bis 2,

wobei das Copolymer auf Vinylbasis (B) ein (Meth)acrylCopolymer ist, das mindestens eine konstitutionelle Einheit, ausgewählt aus der Gruppe, bestehend aus einer konstitutionellen Einheit (u1) mit mindestens einer durch Formel (4), Formel (5) oder Formel (6) dargestellten Struktur (I), einer konstitutionellen Einheit (u2), die eine Triorganosilyloxycarbonylgruppe enthält, und einer konstitutionellen Einheit (u3) mit mindestens einer durch Formel (7) oder Formel (8) dargestellten Struktur (III), einschließt,

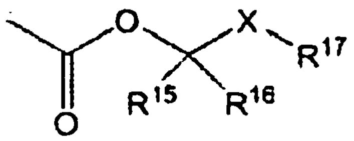
(4)

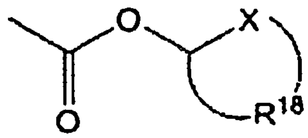
(5)

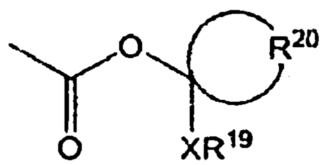
(6)

(in den Formeln stellt X -O-, -S- oder -$NR^{21}$- dar, worin $R^{21}$ ein Wasserstoffatom oder eine Alkylgruppe darstellt, $R^{15}$ und $R^{16}$ stellen jeweils ein Wasserstoffatom oder eine Alkylgruppe mit 1 bis 10 Kohlenstoffatomen dar, $R^{17}$ und $R^{18}$ stellen jeweils eine Alkylgruppe mit 1 bis 20 Kohlenstoffatomen, eine Cycloalkylgruppe oder eine Arylgruppe dar und $R^{18}$ und $R^{20}$ stellen jeweils eine Alkylengruppe mit 1 bis 10 Kohlenstoffatomen dar),

$$-COO-M-OCO\cdots \quad (7)$$

$$-COO-M-R^{13}\cdots \quad (8)$$

(in den Formeln stellt M Zn, Cu, Mg oder Ca dar und $R^{13}$ stellt einen anderen organischen Säurerest als eine (Meth)acryloyloxygruppe dar).

**4.** Antifouling-Farbzusammensetzung gemäß einem der Ansprüche 1 bis 3, ferner umfassend: mindestens eine/eines, ausgewählt aus der Gruppe, bestehend aus einer Verbindung, die mit einer Säure reagiert, einer basischen Verbindung, einer sauren Verbindung und einem Dehydratisierungsmittel.

**5.** Antifouling-Farbzusammensetzung gemäß Anspruch 4,

wobei die Verbindung, die mit einer Säure reagiert, mindestens eine Verbindung (Y3), ausgewählt aus der Gruppe, bestehend aus durch Formel (31), Formel (32) und Formel (33) dargestellten Verbindungen, ist,

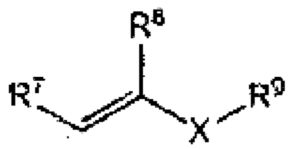
(31)

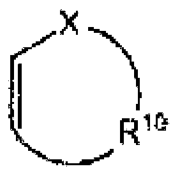
(32)

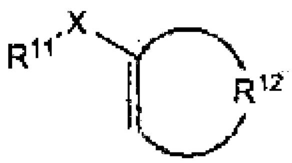
(33)

(in den Formeln stellt X -O-, -S- oder -$NR^{21}$- dar, worin $R^{21}$ ein Wasserstoffatom oder eine Alkylgruppe darstellt, $R^7$ stellt ein Wasserstoffatom oder eine Alkylgruppe mit 1 bis 9 Kohlenstoffatomen dar, $R^8$ stellt ein Wasserstoffatom oder eine Alkylgruppe mit 1 bis 10 Kohlenstoffatomen dar, $R^9$ und $R^{11}$ stellen jeweils eine Alkylgruppe mit 1 bis 20 Kohlenstoffatomen, eine Cycloalkylgruppe oder eine Arylgruppe dar, $R^{10}$ stellt eine Einfachbindung

oder eine Alkylengruppe mit 1 bis 9 Kohlenstoffatomen dar und $R^{12}$ stellt eine Alkylengruppe mit 1 bis 9 Kohlenstoffatomen dar).

**6.** Antifouling-Farbzusammensetzung gemäß einem der Ansprüche 1 bis 5, ferner umfassend ein Antifoulingmittel.

**7.** Antifouling-Farbzusammensetzung gemäß Anspruch 6,
wobei die Antifouling-Farbzusammensetzung als das Antifoulingmittel mindestens eines, ausgewählt aus der Gruppe, bestehend aus Kupfer(I)-oxid, Pyridintriphenylboran, 4,5-Dichlor-2-n-octyl-4-isothiazolin-3-on, 4-Brom-2-(4-chlorphenyl)-5-(trifluormethyl)-1H-pyrrol-3-carbonitril und Medetomidin, enthält.

## Revendications

**1.** Composition de peinture antisalissure, comprenant :

au moins un composé (A) choisi dans le groupe constitué par les composés représentés par la formule (1), la formule (2) et la formule (3) ; et
un copolymère à base de vinyle (B), dans laquelle la teneur en composé (A) dans la composition de peinture antisalissure est de 3 % en masse ou plus, et le rapport massique entre la teneur en composé (A) et le copolymère à base de vinyle (B) dans la composition de peinture antisalissure est de 0,1/99,9 à 70/30,

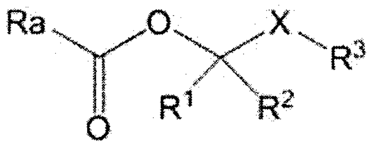
(1)

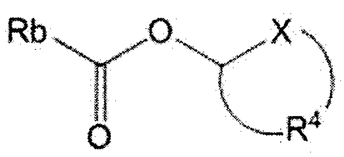
(2)

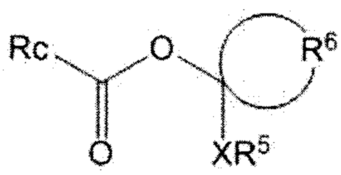
(3)

(dans les formules, Ra, Rb et Rc représentent un groupe hydrocarboné ayant 1 à 40 atomes de carbone ; X représente -O-, -S- ou $-NR^{14}-$, où $R^{14}$ représente un atome d'hydrogène ou un groupe alkyle ; $R^1$ et $R^2$ représentent chacun un atome d'hydrogène ou un groupe alkyle ayant 1 à 10 atomes de carbone ; $R^3$ et $R^5$ représentent chacun un groupe alkyle ayant 1 à 20 atomes de carbone, un groupe cycloalkyle ou un groupe aryle ; et $R^4$ et $R^6$ représentent chacun un groupe alkylène ayant 1 à 10 atomes de carbone).

**2.** La composition de peinture antisalissure selon la revendication 1,
dans laquelle Ra, Rb ou Rc du composé (A) est un résidu hydrocarboné cyclique dérivé de la colophane.

**3.** La composition de peinture antisalissure selon la revendication 1 ou 2,

dans laquelle le copolymère à base de vinyle (B) est un copolymère (méth)acrylique comprenant au moins un motif constitutif choisi dans le groupe constitué par un motif constitutif (u1) ayant au moins une structure (I) représentée par la formule (4), la formule (5) ou la formule (6), un motif constitutif (u2) contenant un groupe triorganosilyloxycarbonyle, et un motif constitutif (u3) ayant au moins une structure (III) représentée par la formule (7) ou la formule (8),

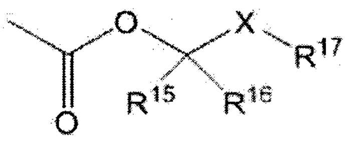
(4)

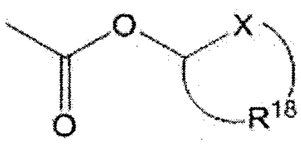
(5)

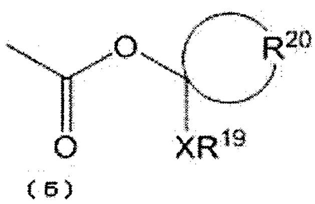
(6)

(dans les formules, X représente -O-, -S- ou $-NR^{21}-$, où $R^{21}$ représente un atome d'hydrogène ou un groupe alkyle ; $R^{15}$ et $R^{16}$ représentent chacun un atome d'hydrogène ou un groupe alkyle ayant 1 à 10 atomes de carbone ; $R^{17}$ et $R^{19}$ représentent chacun un groupe alkyle ayant 1 à 20 atomes de carbone, un groupe cycloalkyle ou un groupe aryle ; et $R^{18}$ et $R^{20}$ représentent chacun un groupe alkylène ayant 1 à 10 atomes de

carbone),

-COO-M-OCO ··· (7)

-COO-M-$R^{13}$ ... (8)

(dans les formules, M représente Zn, Cu, Mg ou Ca ; et R13 représente un résidu d'acide organique autre qu'un groupe (méth)acryloyloxy).

4. La composition de peinture antisalissure selon l'une quelconque des revendications 1 à 3, comprenant en outre : au moins un composé choisi dans le groupe constitué par un composé qui réagit avec un acide, un composé basique, un composé acide et un agent déshydratant.

5. La composition de peinture antisalissure selon la revendication 4,

dans laquelle le composé qui réagit avec un acide est au moins un composé (Y3) choisi dans le groupe constitué par les composés représentés par la formule (31), la formule (32) et la formule (33),

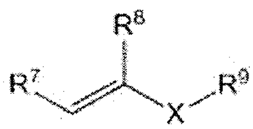
(31)

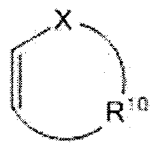
(32)

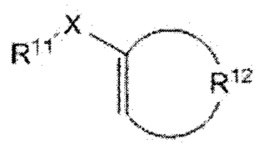
(33)

(dans les formules, X représente -O-, -S- ou -$NR^{21}$-, où $R^{21}$ représente un atome d'hydrogène ou un groupe alkyle ; $R^7$ représente un atome d'hydrogène ou un groupe alkyle ayant 1 à 9 atomes de carbone ; $R^8$ représente un atome d'hydrogène ou un groupe alkyle ayant 1 à 10 atomes de carbone ; $R^9$ et $R^{11}$ représentent chacun un groupe alkyle ayant 1 à 20 atomes de carbone, un groupe cycloalkyle ou un groupe aryle ; $R^{10}$ représente une liaison simple ou un groupe alkylène ayant 1 à 9 atomes de carbone ; et $R^{12}$ représente un groupe alkylène ayant 1 à 9 atomes de carbone).

6. La composition de peinture antisalissure selon l'une quelconque des revendications 1 à 5, comprenant en outre un agent antisalissure.

7. Composition de peinture antisalissure selon la revendication 6,
dans laquelle la composition de peinture antisalissure contient, en tant qu'agent antisalissure, au moins un élément choisi dans le groupe constitué par l'oxyde cuivreux, le du triphénylborane de pyridine, de la 4,5-dichloro-2-n-octyl-4-isothiazolin-3-one, du 4-bromo-2-(4-chlorophényl)-5-(trifluorométhyl)-1H-pyrrole-3-carbonitrile et de la médétomidine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018105921 A
- JP H4103671 A
- JP H1030071 A
- JP H11116858 A
- JP 2001226440 A
- JP 2005082725 A
- EP 3284783 A1
- WO 2017065172 A1
- WO 2018008166 A1
- JP 2001262076 A
- JP 2001261620 A
- WO 2018181429 A1
- JP 2008106047 A
- JP 2018062555 A
- JP 2010100821 A
- JP H06135877 A
- EP 1695956 A1
- EP 1439025 A1
- WO 2013108880 A